# EUROPEAN PATENT APPLICATION

(11) **EP 2 033 577 A1**
(43) Date of publication of application: **11.03.2009**
(21) Application number: 07745090.6
(22) Date of filing: 12.06.2007
(51) Int. Cl.: A61B 5/151

(54) **LANCET ASSEMBLY**

(30) Priority: 13.06.2006 JP 2006163500
(71) Applicant: Izumi-Cosmo Company Limited, Osaka-shi, Osaka 530-0005 (JP)
(72) Inventor: KITAMURA, Yoritaka, Osaka-shi, Osaka 530-0005 (JP); ABE, Teruyuki, Chuo-ku, Tokyo 103-0022 (JP); SEKI, Kazuharu, Setagaya-ku, Tokyo 154-0001 (JP)
(74) Representative: Wiedemann, Peter
(86) International application number: PCT/JP2007/061803
(87) International publication number: WO 2007/145204

(57) **Abstract**

There is provided a lancet assembly with a possibility of the lancet structure being inadvertently pushed without an intension of pricking.

The lancet assembly comprising a lancet structure 200 and a lancet holder 100 which holds the lancet structure, in such a constitution that by pressing the lancet structure into the lancet holder, the following steps are carried out: the step of separating a lancet cover 214 from a lancet body 216 so as expose a distal end portion 232 of a pricking element 220, and then the step of launching the lancet body having the distal end portion of the pricking element exposed so as to prick a predetermined portion, **characterized in that**
a trigger 300 used to launch the lancet body has a pushbutton 117 exposed through an opening 120 provided in a side surface 101 of the lancet holder,
the lancet body of which the distal end portion 232 of the pricking element 220 is exposed is launched by moving the trigger forward while the exposed pushbutton is pressed into the lancet holder.

## Description

### Technical Field

The present invention generally relates to a pricking device such as a finger pricking device or a lancet assembly used to take a small amount of blood sample by pricking skin, and particularly to a lancet assembly of a disposable type that is easy to use.

### Background Art

Various finger pricking devices or lancet assemblies have been commercialized for individual users, as well as for hospitals, clinics and medical practitioners, in order to take a small amount of blood sample. Such devices include a lancet which has an element having a sharp tip portion, namely a pricking element (such as a needle-like element), that is used to quickly prick the skin of a patient, or penetrate the skin so as to form an opening and allow a small amount of blood to bleed.

A sharp distal end portion of such a lancet assembly is sterilized during the manufacturing process, because the pricking element forms a wound. The assembly must be protected from contamination by its ambient environment so as to maintain the sterilized condition until the assembly is put into use. It is also necessary to keep the pricking element from being exposed unnecessarily, in order that the pricking element does not cause injury a human or an object nearby when it is being handled.

With such considerations described above, a lancet assembly has been proposed which comprises a lancet structure wherein a distal end portion of the pricking element is sealed with a resin, and a lancet holder to be used in combination with the lancet structure (refer to Patent Document 1 below).

To use the lancet assembly described above, it is necessary to remove a resin cover, which seals the distal end portion of the pricking element, from the lancet structure inserted in a lancet holder, then hold the lancet holder by pressing, for example, a finger tip onto an opening located at the front end of the lancet holder, and then apply a force toward the finger tip so as to push the lancet structure into the lancet holder, thereby to launch the lancet of the lancet structure.

It is inconvenient to hold the lancet assembly with fingers of one hand and remove the resin cover that seals the distal end portion of the pricking element with fingers of the other hand, when using the lancet assembly described above. Thus it is desired to eliminate the need of the operation to remove the cover.

When the lancet is launched by using the lancet assembly described above, some of the users may be forced to make "mental preparation" in high psychological tension due to the anticipation of experiencing a pain caused by the needle-like pricking element penetrating the skin, for a period lasting from the completion of preparation for launching the lancet to the time when the lancet is launched. It is desired to mitigate such a tension.

Accordingly, a new lancet assembly capable of eliminating the problem of the conventional lancet assembly as described above has been proposed, that makes it unnecessary to remove the resin cover and can remove the psychological tension upon launching (refer to Patent Document 1 below).

This lancet assembly is constituted such that when the lancet structure is pushed by a thumb into the lancet holder which is held between, for example, an index finger and a middle finger so as to continue an action to insert the lancet structure into the lancet holder, the lancet cover is separated from the lancet body so that a distal end portion of the pricking element is automatically exposed, followed by continued action of the insertion operation that leads to automatic launching of the lancet of which the distal end portion of the pricking element is exposed.
[Patent document 1]
International Patent Publication No. WO 2006/038340

### Disclosure of the Invention

### Problems to Be Solved by the Invention

Through studies as to the lancet assembly described in Patent Document 1 that is capable of solving the problems described above, the inventors of the present application have found that, when the lancet structure is inserted into the lancet holder to assemble the lancet assembly or the lancet assembly is picked up while held between fingers, the lancet structure may be inadvertently pushed into without an intension of carrying out the pricking operation, thus resulting in accidental launch of the lancet. Since this type of lancet assembly, once the lancet has been launched, cannot be used again, the lancet assembly that has accidentally launched is wasted. Accordingly, an object of the present invention is to provide a lancet assembly that undergoes the pricking operation only when the user of the lancet assembly intends to do the operation.

### Means to Solve the Problems

Through the studies aiming at achieving the object described above, the inventor of the present application have reached a finding that the problems described above are solved by a lancet assembly having such a constitution that, when inserting a lancet structure into a lancet holder, a lancet cover is automatically removed from a pricking element so as to expose the distal end portion of the pricking element, by applying a force that separates the lancet cover, that surrounds the distal end portion of the pricking element, from a lancet body, and as the operation to push into is further continued, the lancet of which distal end portion is exposed is automatically launched, wherein a trigger that constitutes an ejector of the lancet structure that is inserted is provided with a locking mechanism.

In the first embodiment, the present invention provides a lancet assembly comprising a lancet structure and a lancet holder which holds the lancet structure, in such a constitution that by pressing the lancet structure into the lancet holder, the following steps are carried out preferably continuously (or successively): the step of separating a lancet cover from a lancet body so as expose a distal end portion of a pricking element, and then the step of launching the lancet body having the distal end portion of the pricking element exposed so as to prick a predetermined portion, wherein a trigger used to launch the lancet body has a pushbutton exposed through an opening provided in a side surface of the lancet holder, so that the lancet body of which the distal end portion of the pricking element is exposed is launched by moving the trigger forward while the exposed pushbutton is pressed into the lancet holder.

"One preferred embodiment" of the lancet assembly according to the present invention is characterized in that a front end portion of the pushbutton is abutting against with a wall that defines the front end portion of the opening of the side surface in the state of the pushbutton being exposed.

"Another preferred embodiment" of the lancet assembly according to the present invention is characterized in that the front end portion of the pushbutton is separated from the wall that defines the front end portion of the opening of the side surface in the state of the pushbutton being exposed. In this embodiment, the pushbutton preferably has a flap or flange that protrudes backward from a rear portion of the pushbutton, so that the flap is disposed above a wall that defines a rear end portion of the opening of the side surface in the state of the pushbutton being exposed.

As to the lancet assembly of the present invention described above, that is, the lancet assembly comprising the lancet structure and the lancet holder which holds the lancet structure, in such a constitution that by pressing the lancet structure into the lancet holder, the following steps are carried out preferably continuously (or successively): the step of separating the lancet cover from the lancet body so as expose the distal end portion of the pricking element, and then the step of launching the lancet body having the distal end portion of the pricking element exposed so as to prick the predetermined portion, such lancet assembly may be a lancet assembly as disclosed in International Patent Publication No. WO2006/038340 referred to in the above. Accordingly, in the present specification, reference is made to International Patent Publication No. WO2006/038340, the disclosure of which constitutes a portion of the present specification.

More specifically, the present invention provides a lancet assembly that comprises a lancet structure and a lancet holder which holds the lancet structure, wherein the lancet structure is composed of an ejector and a lancet;
the ejector comprises a trigger, an arm, a spring and a base onto which the trigger, the arm and the spring are mounted;
the spring has a connector provided on its front end and is connected to the base at its rear end;
the lancet comprises a lancet body, a lancet cover and a pricking element with the pricking element being housed in the lancet body and in the lancet cover while straddling across these components;
the distal end portion of the pricking element is enclosed by the lancet cover;
the lancet body is connected to the connector; and
the lancet holder has, at the front end thereof, an opening through which the distal end portion of the pricking element passes.
Such assembly of the present invention is characterized in that when the lancet structure is inserted in the lancet holder and the base is moved relatively toward the connector, the spring is compressed with the connector being engaged with the lancet holder, and also the arm exerts a force to separate the lancet cover and the lancet body from each other, so that the lancet cover is removed from the pricking element and the distal end portion of the pricking element which has been enclosed is exposed, and then when the movement of the base is further continued, the trigger releases the connector from the engagement state. In addition, the assembly of the present invention is characterized in that the trigger has a locking mechanism that acts in cooperation with the lancet holder.

Therefore, in the lancet assembly according to said "one preferred embodiment" of the present invention, it is preferable that in such a state as the lancet structure is incorporated in the lancet holder, the front end portion of the trigger is exposed through the opening provided in the side surface of the lancet holder (preferably exposed while protruding a little from the opening of the lancet holder outward (or upward)) while the pushbutton of which front end portion abuts against the wall that defines the front end portion of the opening of the side surface is provided as the locking mechanism. In this case, the lancet body in which the distal end portion of the pricking element is exposed can be launched by moving the trigger forward while the exposed pushbutton is held being pressed into the lancet holder inside. Therefore, when the pushbutton is not pressed into, the front end portion of the trigger abuts against the wall that defines the front end portion of the opening of the side surface and therefore the lancet structure cannot be moved further forward, and it is restricted or usually becomes substantially impossible to push (or insert, that is, to move forward) the lancet structure further into the lancet holder. In this embodiment, the trigger and the lancet holder are constituted such that the trigger can be moved forward while the exposed pushbutton is pressed into the lancet holder. The forward movement as described above eventually makes it possible to launch the lancet body with the distal end portion of the pricking element being exposed.

Also, in the lancet assembly according to said "another preferred embodiment" of the present invention, it is preferable that in such a state as the lancet structure is incorporated in the lancet holder, the front end portion of the trigger is exposed through the opening provided in the side surface of the lancet holder (preferably exposed while protruding a little from the opening of the lancet holder outward (or upward)) while the pushbutton of which front end portion is separated from the front end wall that defines the front end portion of the opening of the side surface is provided as the locking mechanism. In this embodiment, the lancet button portion (hence the trigger) and the lancet holder are constituted such that the pushbutton cannot be pressed into while the front end portion of the trigger is separated from the wall that defines the front end portion of the opening of the side surface.

In said "another preferred embodiment" described above, it is more preferable that the pushbutton has a flap or flange that protrudes from the rear portion thereof backward, so that the flap is disposed above the wall that defines the rear end portion of the opening of said side surface in the state of the pushbutton being exposed. When the flap is disposed in such disposed state, it is substantially impossible to press the pushbutton into the lancet holder even when a force is exerted in order to push the flap into the lancet holder, because of the existence of the wall that defines the rear end portion of the opening of said side surface. When the pushbutton is moved forward so that the flap moves from the state of being disposed above the wall that defines the rear end portion of the opening of said side surface and the front end portion of the trigger is separated from the front end wall that defines the opening of the side surface toward the wall that defines the front end portion of the opening of said side surface, preferably into abutting against said wall, the flap moves from the state of being disposed above the wall that defines the rear end portion of the opening of said side surface to a position in front of such wall, so that the pushbutton can be pressed into the lancet holder, similarly to the case of said "one preferred embodiment" described above.

In said "another preferred embodiment" described above, when the trigger is moved forward, the distance between the front end portion of the trigger and the front end wall that defines the opening on the side surface that are separated from each other decreases so that the front end portion of the trigger eventually contacts the front end wall that defines the opening of the side surface. As a result, the lancet structure cannot be moved further forward and it is restricted and usually becomes substantially impossible to push (or insert, that is, to move forward) the lancet structure further into the lancet holder.

In this embodiment, too, the trigger and the lancet holder are constituted such that the trigger can be moved forward while the exposed pushbutton is pressed into the lancet holder after the state of contact is achieved as described above. Such a forward movement eventually enables it to launch the lancet body in which the distal end portion of the pricking element is exposed.

The exposed pushbutton can be pressed into the lancet holder, that is, it can move into the lancet holder. As a result, applying a force to the pushbutton in a direction toward the inside of the lancet holder causes the trigger to undergo elastic deformation at least in a part thereof, so that the pushbutton enters the lancet holder and the front end portion of the pushbutton is released from the abutting condition against the front end wall of the opening in the side surface. Thus, when a force is applied to the pushbutton in a direction toward the inside of the lancet holder while a force is applied so as to further push the lancet structure into the lancet holder (namely a downward force), the pushbutton of the trigger, particularly the front end portion thereof moves into the lancet holder so that the lancet structure is released from the state of being restricted with regard to further pushing into thereof, and the trigger, hence the lancet structure, is enabled to move forward within the lancet holder.

The term "pushbutton" is used in the present specification. The part "push" of the term "pushbutton" of course refers to the operation of intentionally applying a force to move the pushbutton toward the inside of the lancet holder when using the lancet assembly of the present invention, although it is preferable that the lancet holder is held between two fingers (for example, an index finger and a middle finger) so that one finger (for example, the index finger) is placed on the pushbutton, which results in or naturally leads to the pushbutton being pushed toward the inside of the lancet holder so that the locked state is released, and in such state, it is possible to operate the lancet assembly for pricking with one hand by depressing the base of the lancet structure with other finger (for example, a thumb) (that is, the lancet body can be launched after the distal end portion of the pricking element of the lancet body has been exposed). With this regard, it is preferable that the pushbutton protrudes outwardly from the side surface of the lancet holder, and in addition, the front end portion of the pushbutton defines a sloped surface that extends downward and forward as will be described later, so that releasing of the locking state and then pushing into of the base can be done smoothly.

Upon using the lancet assembly of the present invention that has the pushbutton in such a state as the front end portion of the trigger is separated from the front end wall that defines the opening of the side surface, when the lancet holder is held between two fingers and a force directed forward is applied with a thumb so as to push the base of the lancet structure into the lancet holder (that is, to move the base forward), the front end portion of the trigger moves toward the front end wall that defines the opening of the side surface so as to abut against the front end wall, thus resulting in temporary halt of the forward movement of the lancet structure. Then, the locked state can be released, similarly to the case described previously, by pressing the pushbutton into the lancet holder while applying a force directed forward with a thumb. That is, the front end portion of the trigger can move forward within the lancet holder, eventually making it possible to launch the lancet.

Such trigger has an elongated form that extends forward as a whole, preferably a strip form, with at least the pushbutton as a whole extends forward obliquely upward with respect to the direction in which the side surface of the lancet holder extends. In addition, in order to make it possible to press the trigger into the lancet holder, it is preferable that the trigger can undergo elastic deformation at least in a part thereof. For example, when the trigger is made of a resin and a portion thereof is made thinner, the trigger can undergo the elastic deformation in the thinned portion.

It is noted that the rear end of the spring may be connected, instead of or in addition to the base, to the arm, preferably to the rear end of the arm or to the vicinity thereof, and more preferably to the base of the arm where the arm is attached to the base or to the vicinity thereof. Also, the rear end of the trigger may be attached, instead of or in addition to the base, to the arm, preferably to the rear end of the arm or to the vicinity thereof, and more preferably to the base of the arm where the arm is attached to the base or the vicinity thereof.

Upon using a lancet assembly according to the one preferred embodiment of the present invention having such a constitution as described above so as to take an amount of a blood sample, when the base of the lancet structure which has been inserted into the lancet holder is pressed, it become impossible to move the lancet structure further forward, since the front end portion of the pushbutton of the trigger is exposed through the opening of the side surface of the lancet holder and such front end portion is already or gets in abutting against the front end wall that defines the opening of the side surface.

Then, when as the pushbutton of the trigger is pressed toward the lancet case so as to release such abutting state (that is, disengage the locking mechanism) and the lancet structure is moved further forward, the connector, and therefore the lancet body is then engaged with the lancet holder so that further movement once becomes impossible while the arm receives a force that moves the lancet structure forward. As a result, while the lancet body is disabled to move forward by the engagement described above, the arm moves forward and therefore a force is applied to the lancet body and the lancet cover so as to separate the lancet body and the lancet cover from each other. In case these members are connected with a weakened portion as will be described later (and as is employed in the known lancet), these members are separated from each other after the weakened portion is broken. As a result, the distal end portion of the pricking element that has been enclosed by the lancet cover is exposed within the lancet holder, so that the opening of the lancet holder is disposed directly in front of the distal end portion of the pricking element. That is, the lancet cover does not lie on the trajectory of the lancet body which has the exposed pricking element that is formed upon the pricking operation. The term "directly" is used in this sense. As a result, the movement of the lancet body is not blocked so that the pricking operation can be carried out. In this way, the distal end portion of the pricking element which has been enclosed by the lancet cover is automatically exposed in the lancet assembly of the present invention. Then, when it is continued to push the base, the trigger of the ejector unlocks the engagement of the lancet body, so that the lancet having the pricking element of which distal end portion is exposed is launched.

In the lancet assembly according to said "another preferred embodiment" of the present invention described above, when the lancet structure is pushed into the lancet holder to assemble the lancet assembly, stopping the pushing action, the pushbutton exposes through the opening provided in the side surface of the lancet holder after the lancet body has been engaged by the lancet holder and the spring has been compressed, and then when pushing into is stopped, the compressed spring expands a little, the locking mechanism may be put into action by the presence of the front end portion of the trigger in a state of being located behind at a distance from the front end wall that defines the opening of the side surface of the lancet holder. In this case, when the pushbutton has a flap or flange that protrudes backward from the rear portion thereof, the flap extends backward beyond the rear end wall that defines the opening of the side surface of the lancet holder.

After pushing the pushbutton of the trigger into the lancet case, canceling the abutting state of the pushbutton and moving the lancet body further forward, engaging of the lancet body with the lancet holder as described above, exposing the distal end portion of the pricking element and the subsequent unlocking of the engagement by the trigger can be carried out successively (in this order) by pushing the base continuously (or successively) into the lancet holder after the locking mechanism is released. Thus, the lancet is automatically launched as the base is pushed into. That is, such a situation can be achieved that the lancet has been launched while the user has been unaware of it. It needs not to say that these operations may also be intentionally carried out intermittently.

In the lancet assembly of one embodiment of the present invention,
the lancet cover is located in front of the arm,
the lancet cover and the lancet body are connected together through a weakened portion,
the connector is connected (or attached) to the lancet body, and
when the base is moved relatively toward the connector (i.e. they get closer to each other) so as to compress the spring while keeping the front end of the arm abutting against the rear end of the lancet cover after releasing the locking mechanism, the lancet cover and the lancet body are separated at the weakened portion. When the lancet cover is moved away from the lancet body after such separation, the distal end portion of the pricking element is exposed which has been enclosed by the lancet cover.

In the lancet assembly of another embodiment of the present invention,
the lancet cover is positioned in front of the arm,
the lancet cover and the lancet body are provided as independent members from each other and are connected together via the pricking element,
the connector is connected to the lancet body, and
when the base is moved relatively toward the connector (i.e. they get closer to each other) so as to compress the spring while keeping the front end of the arm abutting against the rear end of the lancet cover after releasing the locking mechanism, the lancet cover gets away from lancet body. Thereafter, when the lancet cover is moved further away from the lancet body, hence from the pricking element so as to remove the lancet cover from the pricking element, the distal end portion of the pricking element is exposed which has been enclosed by the lancet cover.

When the arm is moved forward while the distal end portion of the pricking element is exposed as described above, the lancet cover which has been separated from the lancet body and is in contact with the front end of the arm moves obliquely forward (for example, forward obliquely upward or forward obliquely downward), so that the opening positioned at the front end of the lancet holder is located directly in front of the pricking element that has been exposed.

In a preferred embodiment of the lancet assembly of the present invention, the front end of the arm engages with the lancet cover. As a result, after the lancet cover has been removed from the pricking element, the abutment state of the lancet cover against the arm is maintained by the arm. For example, the front end of the arm has a hooked portion (or L-shaped portion) that is bent outwardly, and the lancet cover has, on the side thereof, a portion (for example, a hooked portion that is bent inwardly) that engages with the hooked portion. This constitution makes it possible to restrict the lancet cover by means of the arm, which has been separated from the pricking element.

In one embodiment of the lancet assembly of the present invention, the lancet holder has a guiding means provided on a side inner wall at the front end of the lancet holder,
the lancet cover has a guided means which is guided by the guiding means, and
when the guiding means and the guided means cooperate with each other, the lancet cover which has been removed is caused to move forward by the arm which is moving forward and the lancet cover moves obliquely forward (for example, obliquely upward or obliquely downward).

Specifically, the lancet holder has, as the guiding means, a sliding portion that extends obliquely forward along the side inner wall at the front end portion of the lancet holder, and
the lancet cover has, as the guided means, a portion (for example, a protruding portion) which slides on the sliding portion. For example, a part of the hooked portion of the lancet cover may serve as the guided means.

For example, the lancet cover has, as the slid means, a tapered portion which tapers off in the vertical direction toward the front along the side in the front end portion of the lancet holder, and the lancet holder has, as the sliding portion, a tapered portion which flares in the vertical direction along which the tapered portion moves sliding on the lateral face of the inner wall in the front end of the lancet holder (namely, a reverse tapered portion), so that these tapered portions slide relative to each other, thereby causing the lancet cover which has been removed to move obliquely forward.

In another example, the sliding portion may be a protrusion or a recess which has a sliding surface extending obliquely forward provided on the side inner wall of the front end of the lancet holder, and the slid portion may be a protrusion provided on the side of the lancet cover. Such the protrusion is placed on the protrusion provided on the side inner wall of the front end of the lancet holder or fits into the recess and is placed on the sliding surface.

The base, the arm, the spring and the connector of the ejector are formed integrally from a resin, and preferably formed integrally by injection molding of a resin. The trigger may also be formed integrally with the ejector. In a preferred embodiment, the components other than the trigger of the ejector are formed in an integrated member in advance as described above while the trigger is formed in advance a separate member, and the trigger component is combined with the integrally formed member thereby to obtain the ejector. This combining process can be carried out by fitting a portion of the trigger component into the integrally formed member. This fitting process can be done by using a protrusion which can be fitted into, preferably press-fitted into a recess. It is noted that the spring may be an independent member made of a metal, in which case ends of the spring may be connected to the base and the connector respectively. In this case, both the base and the connector have members to which the spring is to be attached.

The lancet body and the lancet cover are preferably formed integrally from a resin as a lancet by inserting the pricking element (typically a needle made of a stainless steel), and preferably formed as a lancet by injection molding. The lancet body and the lancet cover are connected into an integrated piece by a notch (for example, a notch formed from a resin), and it is preferable that the notch functions as a weakened portion. In this case, the lancet body and the lancet cover may be separated from each other, with an intermediate portion of the pricking element being exposed therebetween. Alternatively, the intermediate portion of the pricking element may be covered by a thin resin layer that constitutes the lancet cover and the lancet body and is easily broken (specifically broken with a force comparable to that applied to break the notch). Such a thin layer can be formed when the lancet cover and the lancet body which are connected through the notch are formed by the injection molding.

In other embodiment, the lancet body and the lancet cover may be formed as separate members, with the pricking element included in these members. In this case, the lancet body and the lancet cover may be separated, preferably by a distance as small as possible, while the intermediate portion of the pricking element is exposed between these members. It is noted that the ejector and the lancet are preferably separate members, and these members are preferably connected together through the connector provided on the ejector. In a further embodiment, the ejector and the lancet may be originally formed in a single member, for example, by the injection molding.

The resin that forms the ejector and the lancet, and the lancet holder is preferably one that can be used in the injection molding. Specifically, a polymer material, such as a POM (polyacetal resin), a PBT (polybutylene terephthalate resin), a polyester copolymer resin, an ABS resin, polycarbonate resin, a polystyrene resin, a polyethylene resin or a polypropylene resin may be exemplified.

In the lancet assembly according to said "one preferred embodiment" of the present invention as described above, when the lancet structure is inserted partially into the lancet holder first and the base is moved relatively toward the connector, the front end portion of the trigger is exposed through the opening in the side surface of the lancet holder (preferably exposed while protruding a little from the surface of the lancet holder) and the front end portion of the pushbutton of the trigger abuts against the front end wall that defines the opening of the side surface so that pushing into of the lancet structure into the lancet holder is stopped. That is, the locking mechanism functions (this state may be regarded as the completion of the lancet assembly according to the present invention). Then, when a force is applied to the pushbutton in a direction toward the inside of the lancet holder (upon using the lancet assembly), the trigger is moved forward within the lancet holder, and the spring can be compressed with the connector being engaged with the lancet holder in the lancet holder. That is, the connector is once engaged by the lancet holder, and it is impossible for the connector to move further. Then, when the base is moved further toward the connector, the distance between the base and the connector decreases and the spring is compressed. The state of the connector engaged by the lancet holder can be maintained by the abutment of a portion of the lancet holder against a portion of the connector. In one embodiment, the lancet holder has a stopper, for example a protrusion, provided on the inside thereof, and the connector has a portion, for example a protrusion, which can abut against the protrusion. The action of the locking mechanism and the engagement of the connector may occur in a reverse order.

In the lancet assembly according to said "another preferred embodiment" of the present invention as described above, when the lancet structure is inserted partially in the lancet holder first and a force is applied to the base so as to move relatively toward the connector, the connector is engaged with the inside of the lancet holder, so that the movement of the connector is stopped. Then, when the force is kept applied to the base, the spring is compressed. Then, the front end portion of the trigger is exposed through the opening in the side surface of the lancet holder (preferably exposed while protruding a little from the surface of the lancet holder) and the front end portion of the pushbutton of the trigger abuts against the front end wall that defines the opening of the side surface so that pushing of the lancet structure into the lancet holder is stopped. That is, the locking mechanism functions. Then, when the force applied to the base is removed, the compressed spring expands a little, so that the pushbutton retracts backward and abuts against the rear end wall that defines the opening in the side surface of the lancet holder. At this state, when the pushbutton has a flap or flange that protrudes from the rear portion thereof backward, the flap extends backward beyond and over the rear end wall that defines the opening of the side surface of the lancet holder. This state may be regarded as the completion of the lancet assembly of the present invention.

Then, when a forward force is applied to the base so as to move forward for using the lancet assembly, the front end portion of the pushbutton abuts against the front end wall that defines the opening of the side surface of the lancet holder, so that the forward movement of the base is temporarily stopped. Then, similarly to the lancet assembly of the said "one preferred embodiment" as described above, a force is applied to the pushbutton in a direction toward the inside of the lancet holder and the trigger is moved forward within the lancet holder, so that the spring can be compressed with the connector being engaged with the lancet holder in the lancet holder. The subsequent processes are substantially the same as those described above.

Then, when the base is continued to be moved, the spring is further compressed and the arm moves forward while the lancet body is engaged with the arm, in any of the lancet assemblies of the preferred embodiments. Accordingly, the lancet cover eventually is removed from the pricking element and the distal end portion of the pricking element that has been enclosed by the lancet cover is exposed. Thereafter, the lancet cover moves obliquely forward. As a result, the opening of the lancet holder is positioned directly in front of the distal end portion of the pricking element.

Then, when the base is continued to be moved, the trigger of the ejector, particularly the distal end portion of such trigger, unlocks the state of engagement of the connector, and therefore of the lancet body as described above. The above described state of engagement is a state of a portion of the lancet holder abutting against a portion of the connector, namely a state in which they are pressing each other in the opposite directions (forces acting against each other along the same axis), and therefore the state of engagement can be easily unlocked by bringing the force axes out of alignment. For example, the trigger acts so as to bring the direction, in which the portion of the connector applies the force, out of alignment with the direction in which the portion of the lancet holder applies the force. Specifically, the connector and the lancet holder are constituted such that the connector has a protrusion extending (upward) obliquely upward as said portion thereof, and such protrusion abuts against a protrusion formed as said portion of the lancet holder. When the trigger moves forward toward the protrusion of the lancet holder, it abuts against the obliquely extending protrusion of the connector. Then, when the movement of the trigger is further continued, the trigger that is moving forward presses downward the protrusion of the obliquely extending connector, so that the obliquely extending protrusion is gradually displaced downward, thereby eventually unlocking the state of engagement.

In the lancet assembly of the present invention, the trigger is an elongated member as a whole that extends forward, preferably an elongated plate, rod or bar, and has, as a front portion thereof, the pushbutton that extends forward obliquely upward as a whole. The trigger functions as the locking mechanism in cooperation with the opening in the side surface of the lancet holder and, by moving in the direction of pricking after the locking mechanism has been inactivated, the trigger presses down the protrusion of the connector with respect to the protrusion of the lancet holder, which have been abutting against each other while pressing each other in the pricking direction and in its opposite direction, namely causes a downward movement of the protrusion of the connector in a direction perpendicular to the pricking direction.

When such trigger cancels the state of engagement, the spring is released from its compressed state and instantaneously expands, so as to drive the lancet body to move forward at once, in other words, the lancet body is launched which has the pricking element of which distal end is exposed. In the case wherein a finger is placed on the opening positioned at the front end of the lancet holder at this time, the finger is pricked by the distal end portion of the pricking element. It is noted that the state of the connector being engaged is none other than the state of the lancet body that is connected to the connector being engaged. Therefore, such a constitution may also be employed that the lancet body, instead of the connector, is engaged. For example, the lancet body may be provided with a protrusion formed, preferably at a position near the rear end thereof. In a further embodiment, the protrusion which is to abut may be provided on the spring, particularly near the front end thereof, instead of on the connector.

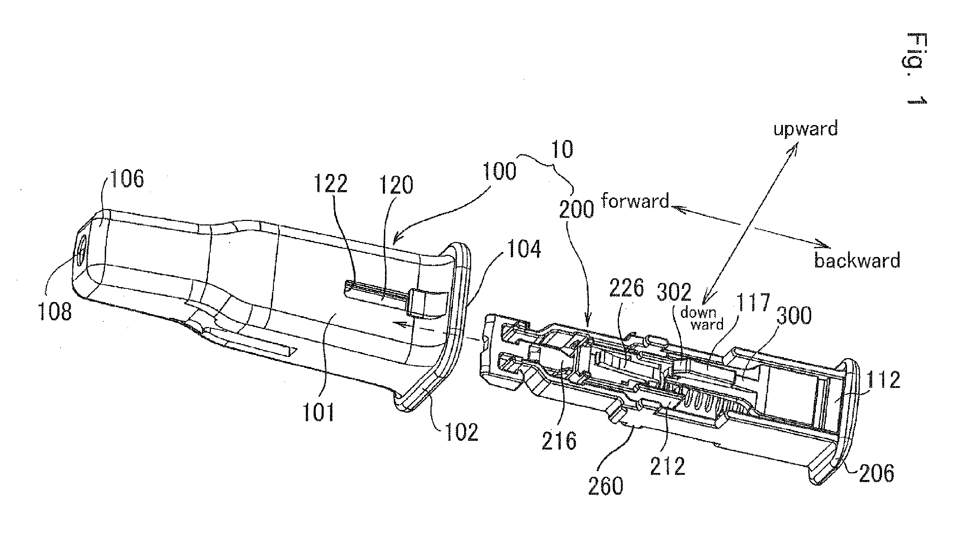

In the present specification, the term "front" (or rear)" is used with reference to the direction in which the pricking element moves for the pricking operation. The term "upward (or downward)" is used in such a manner as the direction along which the lancet cover moves obliquely to expose the distal end of the pricking element is referred to as upward, and the opposite direction is referred to as downward, with reference to the plane which is defined by the two arms shown in the drawing (namely the plane which includes the straight lines along which the arms extend, assuming that the two arms extend in straight lines) and which includes the direction along which the pricking element moves (refer to Fig. 1). The direction which is perpendicular to the front-rear direction and the up-down direction, and which together with these directions constitutes the orthogonal coordinate system will be referred to as the right-left direction.

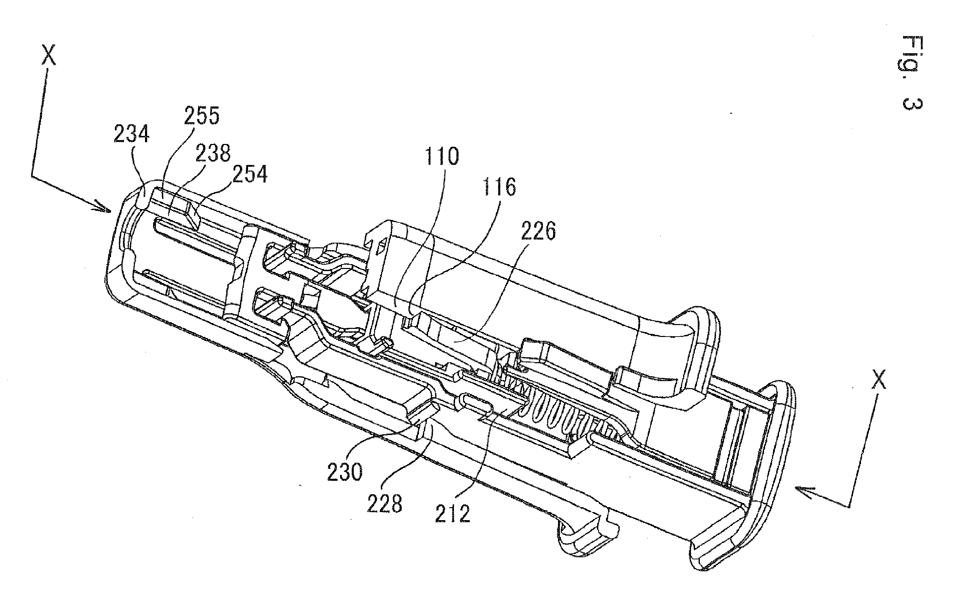

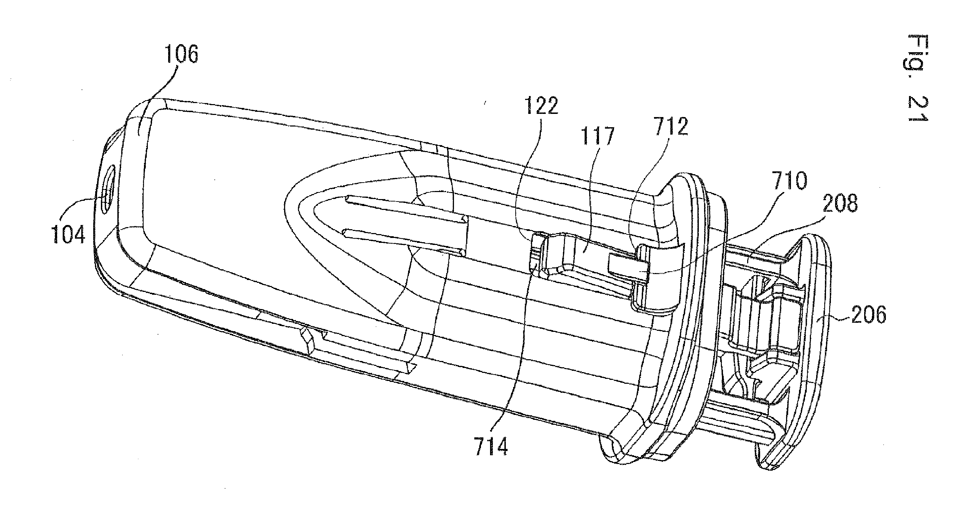

The lancet assembly of the present invention is preferably supplied in a arrangement in the state wherein the lancet structure and the lancet holder described above or to be described later are properly combined. For example, a state wherein at least a portion of the lancet structure is inserted into the lancet holder. In a more preferred embodiment, a whole of the lancet of the lancet structure is inserted into the lancet holder, and the connector is engaged with the lancet holder, in the state of the locking mechanism described above and to be described later being activated so that the connector is engaged with the lancet holder, for example the lancet structure and the lancet holder are combine to reach a state wherein the protrusion of the connector abuts against the inner protrusion of the lancet holder (the state shown in Fig. 3, where the spring may not be compressed, or may be compressed a little, Fig. 3 showing the latter case), or a state wherein the front end portion of the pushbutton is separated from the front end wall that defines the opening of the side surface (state shown in Fig. 21), that is, the state where the lancet assembly of the present invention described above has been completed. In this case as will be described later with reference to Fig. 3, it is particularly preferable that the lancet structure cannot be easily drawn out of the lancet holder. In other embodiment, the lancet structure and the lancet holder are not combined, in which case the present invention may be called a kit of a lancet assembly constituted from the lancet structure and the lancet holder, rather than the lancet assembly.

In the second aspect, the present invention provides the lancet holder and the lancet structure that constitute the lancet assembly described above or to be described later. The present invention also provides the lancet and the ejector which form the lancet structure described above. Descriptions about the lancet assembly of the present invention described above or to be described later are applied also to the lancet holder, the lancet structure, the lancet and the ejector.

### Effects of the Invention

In the lancet assembly of the present invention, a possibility of the lancet structure being inadvertently pushed without an intension of pricking, which results in launching of the lancet is significantly reduced, since launching of the lancet requires to inactivate the action of the locking mechanism with an intension of pricking.

### Brief Description of the Drawings

Fig. 1 is a schematic perspective view of the lancet assembly according to said "one preferred embodiment" of the present invention before the lancet structure is inserted into the lancet holder.
Fig. 2 is a schematic perspective view of the lancet structure that constitutes the lancet assembly according to said "one preferred embodiment" of the present invention.
Fig. 3 is a schematic perspective view of the lancet assembly according to said "one preferred embodiment" of the present invention in the state that the lancet structure is inserted into the lancet holder and the lancet structure is restricted by the locking mechanism from moving forward.
Fig. 4 is a schematic perspective view of the lancet assembly according to said "one preferred embodiment" of the present invention in the state that the locking mechanism is unlocked in the state shown in Fig. 3, so that the lancet cover is separated from the lancet body.
Fig. 5 is a schematic perspective view of the lancet assembly according to said "one preferred embodiment" of the present invention in the state that the lancet structure is inserted further in the state shown in Fig. 4, so that the lancet cover has been completely separated from the lancet body.
Fig. 6 is a schematic perspective view of the lancet assembly according to said "one preferred embodiment" of the present invention in the state that the lancet structure is inserted further into lancet holder in the state shown in Fig. 5, so that the lancet cover has moved forward obliquely upward.
Fig. 7 is a schematic perspective view of the lancet assembly according to said "one preferred embodiment" of the present invention in the state that the lancet cover has been moved fully upward in the state shown in Fig. 6 and held in the front end of the lancet holder.
Fig. 8 is a schematic perspective view of the lancet assembly according to said "one preferred embodiment" of the present invention in the state that the lancet body is released from the restriction in the state shown in Fig. 7 so that the distal end portion of the pricking element protrudes from the opening positioned at the front end of the lancet holder.
Fig. 9 is a schematic perspective view of the lancet assembly according to said "one preferred embodiment" of the present invention in the state that the spring has restored its original shape in the state shown in Fig. 8, and the distal end portion of the pricking element has retracted backward from the opening positioned at the front end of the lancet holder to the inside.
Fig. 10 is a schematic cross sectional view along line X-X in Fig. 3.
Fig. 11 is a schematic cross sectional view of the state shown in Fig. 4, similarly to Fig. 10.
Fig. 12 is a schematic cross sectional view of the state shown in Fig. 5, similarly to Fig. 10.
Fig. 13 is a schematic cross sectional view of the state shown in Fig. 6, similarly to Fig. 10.
Fig. 14 is a schematic cross sectional view of the state shown in Fig. 7, similarly to Fig. 10.
Fig. 15 is a schematic cross sectional view of the state shown in Fig. 8, similarly to Fig. 10.
Fig. 16 is a schematic cross sectional view of the state shown in Fig. 9, similarly to Fig. 10.
Fig. 17 is a schematic perspective view showing a trigger element that enables it to constitute the ejector by fitting in a base.
Fig. 18 is a schematic perspective view showing the relationship of components that constitute the lancet structure.
Fig. 19 is a schematic perspective view of the lancet assembly according to said "another preferred embodiment" of the present invention before the lancet structure is inserted into the lancet holder.
Fig. 20 schematically shows the lancet assembly according to said "another preferred embodiment" of the present invention in the state that a lancet structure 700 is inserted up to a portion ahead of the front end portion of the trigger, when viewed sideways with respect to the lancet assembly.
Fig. 21 is a schematic perspective view of the lancet assembly according to said "another preferred embodiment" of the present invention in the state that the lancet structure has been further inserted in the state shown in Fig. 20, thereby completing the assembly.
Fig. 22 is a schematic perspective view of the lancet assembly according to said "another preferred embodiment" of the present invention shown in Fig. 21 in the state of being cut away similarly to Fig. 3.
Fig. 23 is a schematic perspective view showing a state wherein the front end portion of the pushbutton is abutting against a wall that defines the front end portion of the opening of the side surface similarly to Fig. 3 by pushing the base upon using the lancet assembly according to said "another preferred embodiment" of the present invention.

### [Description of Reference Numerals]

- 10: Lancet assembly
- 100: Lancet holder
- 101: Side surface
- 102: Rear end portion
- 104: Opening
- 106: Front end portion
- 108: Opening
- 110: Trigger counterpart
- 112: Rear end portion
- 117: Pushbutton
- 118: Sloped surface
- 120: Opening
- 122: Front end portion
- 130: Abutting portion
- 200: Lancet structure
- 202: Ejector
- 204: Lancet
- 206: Base
- 208: Arm
- 210: Spring
- 212: Connector
- 214: Lancet cover
- 216: Lancet body
- 217: Protrusion
- 218: Weakened portion
- 221: Cut-into portion
- 219: Protrusion
- 220: Pricking element
- 222: Front end portion
- 223: Hooked portion
- 224: Rear side
- 225: Space complementary to hooked portion
- 226: Protrusion
- 227: Thin resin layer
- 228, 230: Protrusion
- 232: Distal end portion
- 234: Front wall
- 236: Tapered portion
- 238: Guiding means (Reverse tapered portion)
- 240: Protrusion
- 250: Stepped portion
- 254: Sloped surface, Base end
- 254: inclined surface
- 256: Front portion of lancet body
- 260, 262: Protrusion
- 270: Protrusion
- 272: Space
- 280: Protrusion
- 272: Recess
- 273: Protrusion
- 274: Protrusion
- 300: Trigger
- 302: Front end portion
- 303: Leg
- 304: Trigger element
- 306: Recess
- 310: Elastically deforming portion
- 360: Assembly composed of spring, connector and lancet
- 500: Lancet assembly
- 600: Lancet holder
- 700: Lancet structure
- 710: Flap
- 712: Rear end portion
- 714: Space

### Best Mode for Carrying Out the Invention

A lancet assembly 10 of the present invention is shown in a schematic perspective view of Fig. 1. The lancet assembly 10 is the lancet assembly according to said "one preferred embodiment" described above, and is constituted from a lancet holder 100 and a lancet structure 200. In the embodiment illustrated, the lancet structure 200 is shown in the state immediately before being inserted into the inner space of the lancet holder 100 in the direction indicated by the broken line arrow. It is noted that directions of "forward", "backward", "upward" and "downward" as used in this specification are shown in Fig. 1 (based on the orthogonal coordinate system).

The lancet holder 100 has an opening 104 (not shown in Fig. 1) in the rear end portion 102 thereof, and also has an opening 108 in the front end portion 106. With a part (for example, a finger tip) to be pricked being applied to the opening 108, the part is pricked when the distal end portion of the pricking element which is exposed is ejected out through the opening 108. The lancet structure 200 has a trigger 300 provided on the upper side thereof. The trigger 300 is connected at the rear end 112 thereof to a base 206 of the ejector 202, and has a pushbutton 117 as its front portion. When the lancet structure is inserted and pushed into the lancet holder, the pushbutton 117 of the trigger 300 is exposed through an opening 120 formed in the side surface 101 of the lancet holder 100, and the front end portion 302 of the pushbutton 117 is designed to abut against the wall that defines the front end portion 122 of the opening 120.

In the state where the trigger 300 is connected to the base 206, the trigger 300 is adapted such that at least its button portion 117 extends obliquely upward with respect to the side surface of the lancet holder, so that the front end portion 302 of the pushbutton 117 abuts against the wall that defines the front end portion 122 of the opening 120. When a force is applied to the pushbutton 117 toward the inside of the lancer holder, at least a portion of the trigger 300 is deformed so that the pushbutton 117 enters the lancet holder inside. This deformation preferably occurs elastically, and for example, I is preferable that at least a portion of the trigger, preferably substantially the entire trigger is made of a resin, so that the button portion 117 that extends obliquely upward can be pressed down against the elastic force of the resin. Specifically, when a portion of the trigger 300 (for example, a base portion 310) is formed into a thin and/or narrow resin portion so as to easily undergo the elastic deformation, then the pushbutton 117 elastically moves downward (that is, toward the inside of the lancet holder) when a force is applied toward the inside of the lancet holder due to the elastic deformation of that portion.

In case the pushbutton 117 is exposed through the opening 120 as described above, it is preferable that at least a portion of the pushbutton protrudes upward through the side surface 101, that is, it is in the form of a bulge, which makes being pressed that is carried out subsequently easier. When in such state, the pushbutton 117 is pressed into and the base is pushed forward, the abutting state is canceled so that the trigger 300 can move forward. Thus, the trigger 300 is adapted so as to be capable of moving along the inner wall of the lancet holder (forward in Fig. 1).

It is preferable that front end portion 302 of the pushbutton 117 defines the sloped surface 118 that extends down forward as illustrated, so that the trigger can easily move along the inner wall of the lancet holder after it has been pushed into.

It is preferable that the front end portion 302 has a sloped surface 304 that extends down forward as illustrated, so that the trigger can move smoothly forward when it is pushed into the lancet holder to cancel the state of abutment. The lancet holder 100 has, in the upper side wall thereof, a trigger counterpart 110 which is a portion oppose to the trigger 300, and a portion of the counterpart forms a protrusion 116 that protrudes toward the inside of the lancet holder. A connector 212 to be described later, specifically a distal end of a protrusion 226 that extends obliquely forward from the connector 212 abuts against such protrusion 116, so as to achieve the state of engagement that blocks the connector 212, therefore the lancet body 216 that is connected to the connector from moving forward. This state of blocking can be canceled by pressing the front end portion 302 of the trigger 300 toward the abutting position.

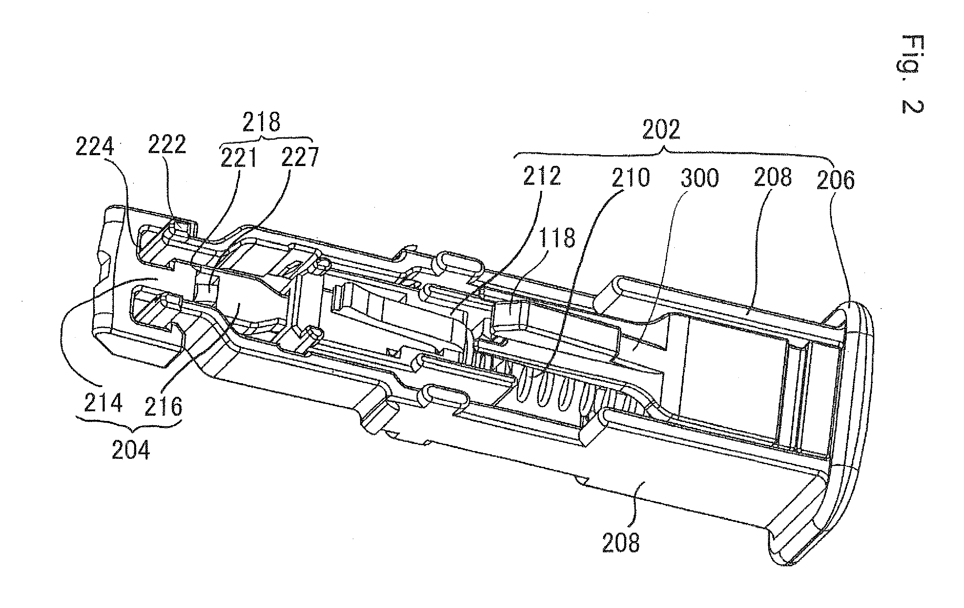

Fig. 2 is a schematic perspective view of the lancet structure 200 shown in Fig. 1. The lancet structure 200 is constituted from an ejector 202 and a lancet 204. The ejector 202 has a base 206 with arms 208 attached on both sides thereof. While it is preferable that a spring is provided between a pair of the arms as illustrated, the number of arms may be 1, 3 or more. A spring 210 of a coil configuration is provided between the arms, and one end thereof is attached to the base 206.

In the embodiment illustrated, the spring 210 is connected to the base 206, although not shown in Fig. 2. The end of the spring 210 may be connected to both of the arms and the base, depending on the form of the spring.
For example, if a spring made of a resin in an undulating configuration is used, it may be connected as just described above. Attached to the other end of the spring 210 is a connector 212. In one preferred embodiment, the base 206 and the arm 208 are formed integrally, while the spring 210 and the connector 212 are formed as independent members, and these members are assembled together. For example, the base and the arm can be formed integrally and the connector can be formed as an independent member by injection molding of resin.

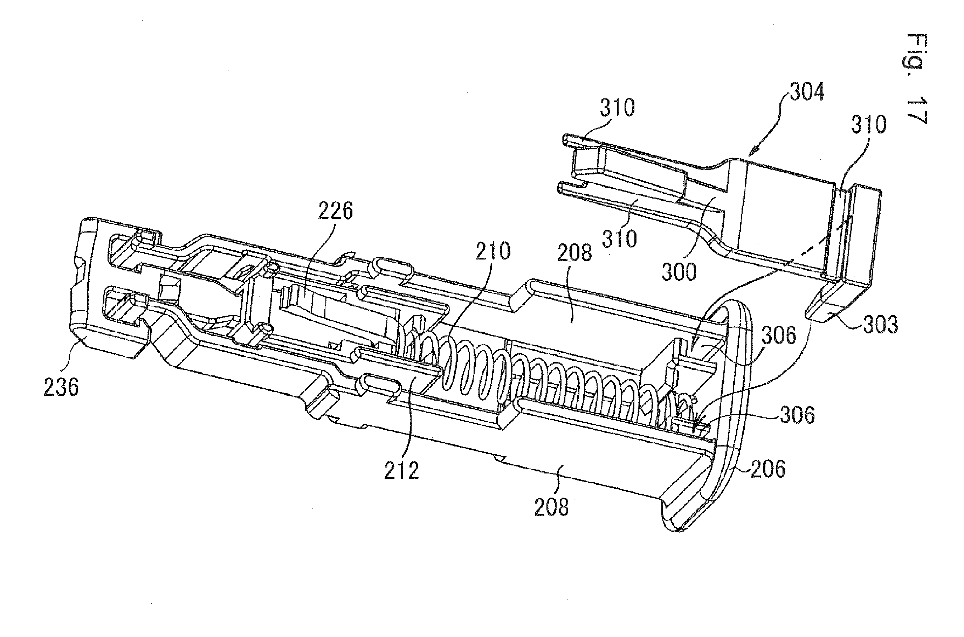

In other embodiment, the base, the arms, the spring and the connector can be formed integrally by molding a resin. The trigger 300 is preferably formed by combining the trigger element that is formed separately with other members that are integrally formed for the formation of the ejector. Specifically, a trigger element 304 having legs 303 is formed in advance as shown in Fig. 17. The base 206 of the ejector is provided with recesses 306 in the base of the arm 208 which recesses are complementary to the protrusions of the legs. It is preferable to constitute the ejector of the present invention by fitting the protrusions 303 into the recesses 306, preferably by press fitting, as indicated by arrows.

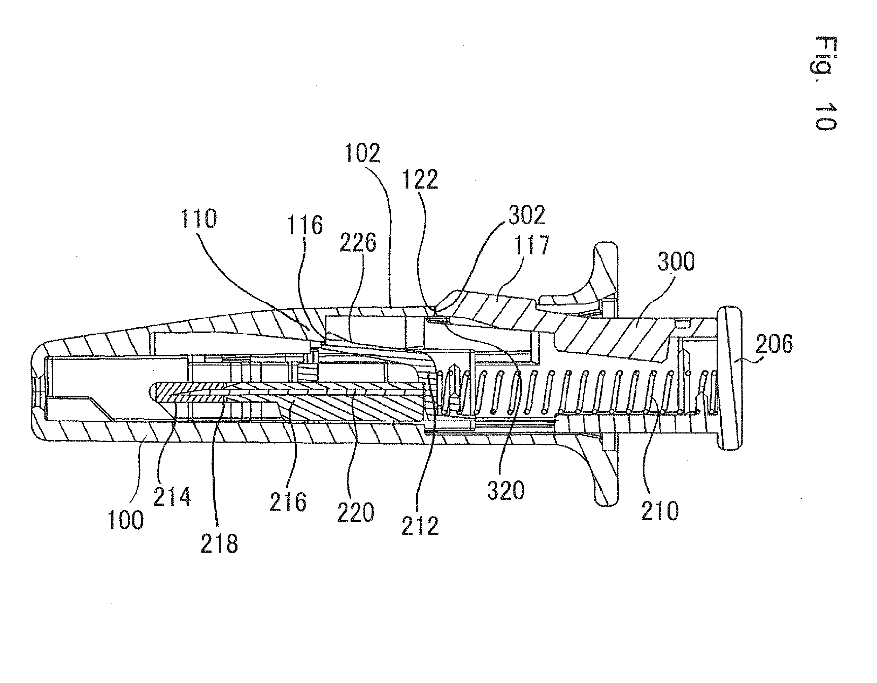

It is preferable that the trigger 300 has an extensions 320 on the sides thereof as illustrated. While the pushbutton 117 extends obliquely upward, the extensions 320 extend substantially horizontally (that is, in the direction in which the side surface 101 of the lancet holder extends). As a result, when the lancet structure 200 is inserted into the lancet holder 100, the pushbutton 117 is exposed through the opening of the lancet holder, while the extensions 320 are not exposed through the opening of the lancet holder and can be located on the lower side of the wall that defines the opening. When such extensions 320 are provided, the extensions contact (or come substantially into contact with) the inner wall of the lancet holder when the lancet structure is inserted into the lancet holder so as to expose the pushbutton 117 through the opening, and therefore the pushbutton is prevented from accidentally popping out of the opening (refer to Fig. 10). The extension 320 may be either disposed to extend from the side of the trigger 300 as illustrated, or formed in an elongated configuration that extends from the base of the trigger 300 forward in parallel to the trigger 300. It is preferable that the front end of the extension 320 extends forward beyond the front end portion 302 of the pushbutton 117 as illustrated.

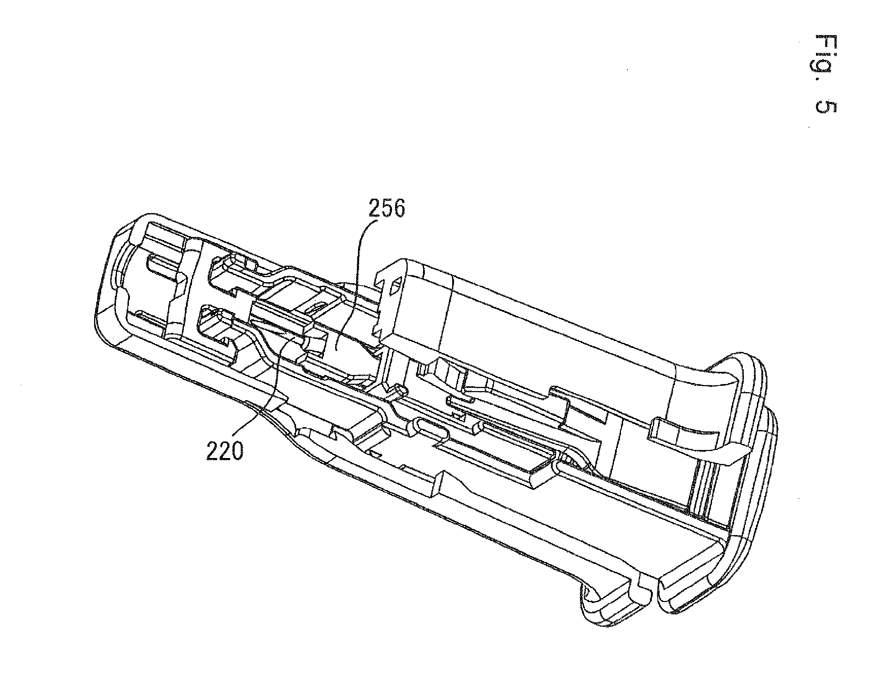

The lancet 204 is constituted from the lancet body 216 and the lancet cover 214, which are connected to each other through a weakened portion 218. The weakened portion 218 may have any proper form. In the illustrated embodiment, for example, the weakened portion 218 is constituted from a cut-into portions 221 of resin portions that connect the lancet body 216 and the lancet cover 214 and a thin resin layer 227 that encloses the pricking element, which will be described later. The cut-into portion may be replaced with a notch (for example, a V-shaped recess). The lancet 204 further comprises a pricking element 220 (refer to Fig. 5), while the distal end portion of the pricking element 220 is enclosed by the lancet cover 214 and is thereby sealed. The rear portion of the pricking element 220 is disposed within the lancet body 216. While a portion of the pricking element 220 may be exposed between the lancet body 216 and the lancet cover 214, in the embodiment illustrated such a portion is also covered by a resin, and at least a portion of which has a form of a thin layer so as to be easily broken when a force is applied by fingers so as to separate the lancet body and the lancet cover from each other. In other embodiment, the lancet body and the lancet cover may be separated from each other as independent members without the weakened portion provided therebetween. The distance of separation between them is preferably as small as possible, preferably nearly zero.

As will be understood from the drawing, the lancet cover 214 is positioned in front of the arm 208 and, in the embodiment shown, the front end portions 222 of the arms 208 are positioned proximately to the rear side 224 of the lancet cover 214. In other embodiment, these members may be in the abutment state against each other (i.e. contact state) without being separated (i.e. without being close to each other). In the embodiment in which these members are disposed close to each other, too, the front ends 222 of the arms 208 abut against the rear side 224 of the lancet cover 214 when the arms 208 are moved further forward after the forward movement of the lancet body 216 has stopped, as will be described later.

The state of the lancet structure 200 being inserted in the state shown in Fig. 1 into the lancet holder 100 so that the button portion 117 of the trigger 300 is exposed through the opening 120 formed in the side surface 101 of the lancet holder is shown in the schematic perspective view of Fig. 3. It is noted that in order to make it easier to understand the state of the lancet structure disposed in the lancet holder 100, the lancet holder is shown in the drawing as partially cut away leaving the lower portion (about a half of the lancet holder) and the rear portion of the distal side half of the upper portion of the lancet holder. In the state shown in Fig. 3, the front end portion 302 of the pushbutton 117 abuts against the wall surface that defines the front end portion 122 of the opening 120.

In the state shown in Fig. 3, the protrusion 226 that extends forward obliquely upward from the connector 212 is abutting against the protrusion 116 that extends downward or downward and backward from the trigger counterpart 110 of the lancet holder 100. This abutting state may be achieved either before the pushbutton 117 is exposed through the opening 120 or simultaneously therewith, or when the base 206 is further pushed after pushing the pushbutton 117 into, as will be described later.

In the embodiment shown, the protrusion 228 having a taper-like shape of which width in the horizontal direction (the direction which is perpendicular to the front-rear direction and the up-down direction in Fig. 1) increases (or flares) toward the distal end is provided on the side of the inner wall of the lancet holder, so that in the course of inserting the lancet structure 202, a wing or flap (preferably having a thin layer form) 230 which extends from the arm obliquely backward provided at a certain position of each arm 208 can move forward while getting over the protrusion 228. The protrusion 228 is positioned preferably such that the protrusion 226 just abuts against the protrusion 116 just when the wing 230 gets over the protrusion 228. In other embodiment, the wing may get over the protrusion 228 before or after abutting. A taper-like shape protrusion (thinning toward the distal end) may also be used instead of the wing 230.

Use of such wing configuration enables it to make use of the elasticity of the wing material (for example, resin material), and makes the wing 230 get over the protrusion 228 easily while, after getting over, making it substantially impossible for the wing 230 to move back by getting over the protrusion 228. It may be advantageous to use the taper-like shape protrusion described above which gives a stronger snapping touch than use of the wing when getting over the protrusion and therefore allows the person who is assembling the device or the user thereof to recognize that the abutment has been achieved. Such a movement of getting over can also be achieved by forming the lancet structure and the lancet holder, particularly the protrusions thereof, from a resin and taking advantage of the elasticity of the resin. Similarly, use of the taper-like shape protrusion makes it substantially impossible for the protrusion 230 to move back by getting over the protrusion 228, once having got over the protrusion 228. As a result, once the state shown in Fig. 3 is achieved, it becomes difficult to draw out the lancet structure from the lancet holder, as described previously. In either case, it becomes substantially impossible to disassemble (namely to draw out the lancet structure from the lancet holder) after setting up or using the lancet assembly, so that reuse and mishandling of the assembly are avoided so that its safety is conveniently ensured.

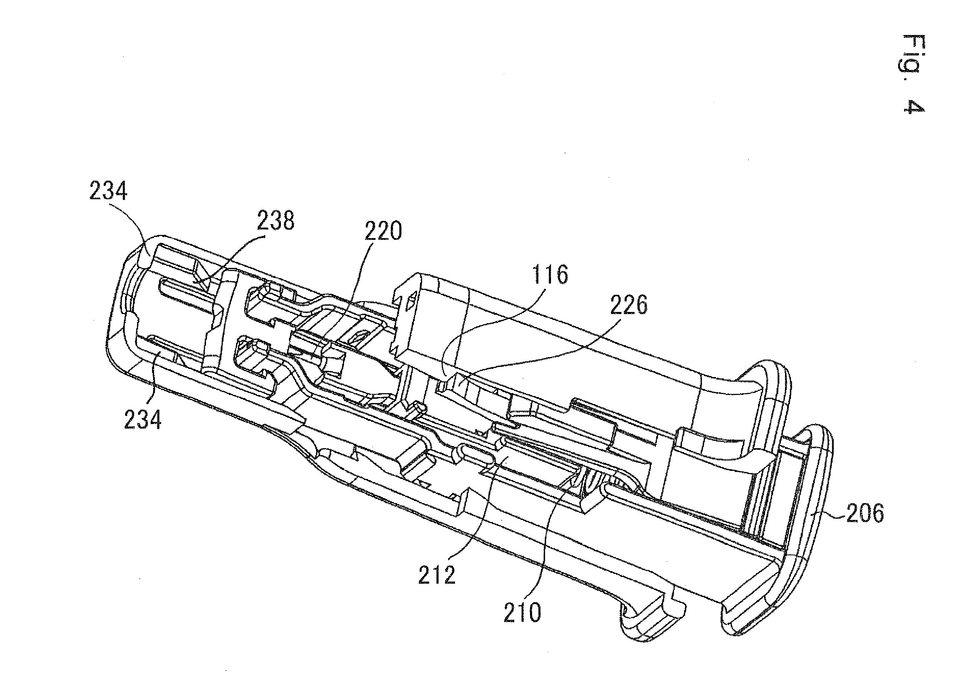

In the state shown in Fig. 3, when the pushbutton 117 is pressed into the lancet holder inside (that is, a force is applied downward to the pushbutton), the pushbutton 117 undergoes downward elastic movement. As a result, the state of abutment between the wall that defines the front end portion of the opening 120 of the lancet holder and the front end portion 117 of the pushbutton is canceled. At this time, when a force is exerted to push the base 206 forward, the trigger 300 is further pushed into at the same time as the state of abutment is canceled, so that the lancet structure 200 is further pushed into. In other embodiment, when the base is pushed into after pressing into, the trigger 300 is further pushed into after the state of abutment is canceled. The state of the lancet structure 200 being pushed into is shown in Fig. 4.

As will be apparent by comparing with Fig. 3, in Fig. 4, the spring 210 has been compressed and also the base 206 has moved forward and the arms 208 have also moved forward, although the position of the connector 212 remains the same as that in Fig. 3 since the connector 212 cannot move forward due to the abutment between the protrusion 226 thereof and the protrusion 116 of the lancet case. Also because the front end portion 302 of the pushbutton 117 of the trigger 300 defines the sloped surface 118 which inclines downward toward the front, it is made easier to start the movement of the pushbutton along the inside of the upper wall of the lancet holder after being pressed into as described above.

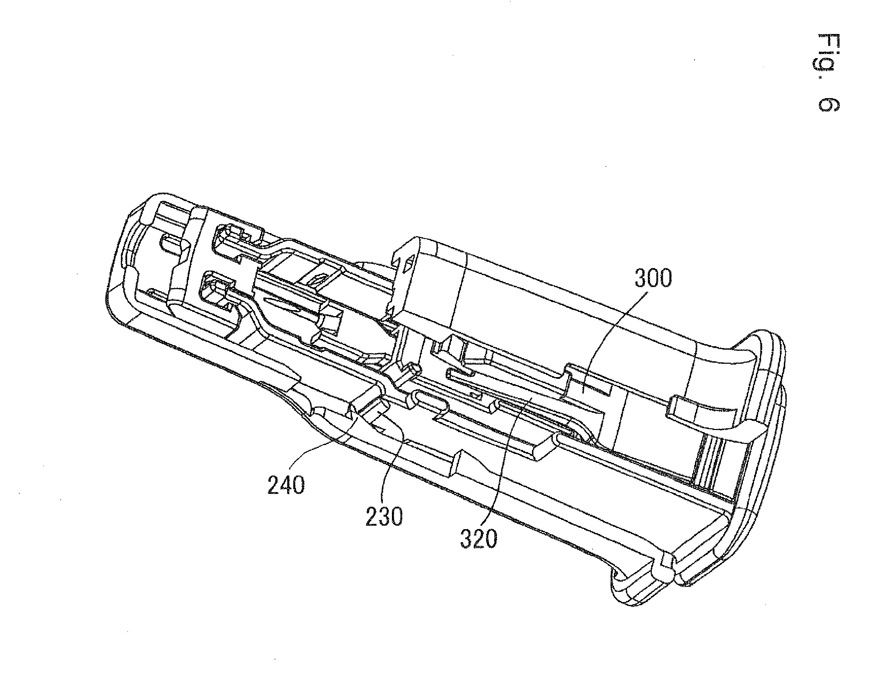

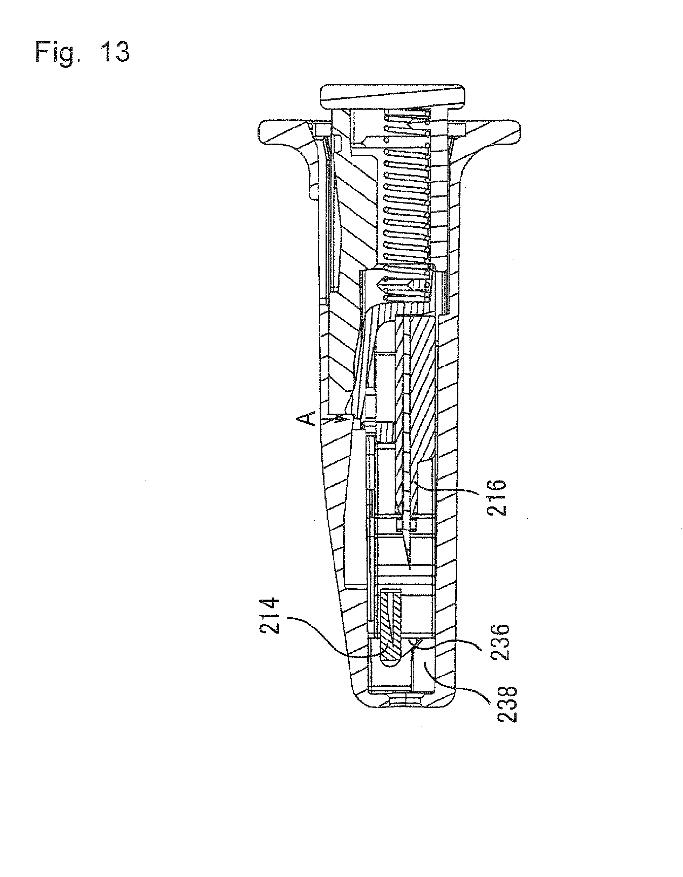

In the state shown in the drawing, the protrusion 116 and the protrusion 226 receive forces that press against each other in the opposite directions along the same line. Therefore, when the base is pushed further in this state so as to cause the trigger 300 to move further forward in the lancet holder, the front end portion 302 of the trigger 300 makes contact with the protrusion 226 which extends obliquely upward as shown in Fig. 6. Then, when the trigger is moved further forward, the front end portion 302 presses downward (refer to the arrow in Fig. 13) the protrusion 226 of the connector which extends obliquely, so that the pressing forces are brought out of the alignment thereby causing the protrusion 226 which extends obliquely upward from the connector 212 is gradually moved downward.

When the lancet structure 200 is pressed further from the state shown in Fig. 3 as described above, the front end portions 222 of the arms 208 abut against the rear side 224 of the lancet cover 214. Then, the arms 208 apply such forces to the lancet cover 214 that intend to cause the lancet cover 214 to move forward. On the other hand, the connector 212 is unable to move forward due to the abutment between the protrusions 116 and 226. Thus, forces act so as to separate the lancet cover 214 and the lancet body 216 that is connected to the connector 212 from each other. As a result, the thin resin layer of the weakened portion 218 between the lancet cover 214 and the lancet body 216 is broken and these members are moved apart, into the state shown in Fig. 4. In the state illustrated, the lancet cover 214 has been moved forward after the breakage, so that distal end portion of the pricking element 220 can be seen.

Then, when the lancet structure 200 is further pushed from the state shown in Fig. 4, the distal end portion 232 of the pricking element 220 is completely exposed as shown in Fig. 5. It is noted that in the embodiment in which the lancet body and the lancet cover are the independent members, the lancet body is moved away from the lancet cover so that the distal end portion of the pricking element is eventually exposed.

Fig. 6 shows a schematic perspective view of the state of applying a force to the base 206 so as to move the lancet structure 200 forward so that the lancet structure 200 is further inserted from the state shown in Fig. 5 in which the distal end of the pricking element is exposed. Fig. 6 shows the state immediately after the front end portion 302 of the trigger 300 which has been moved forward by inserting the lancet structure 200 has pressed the protrusion 226 of the connector downward, thereby bringing the protrusion 226 and the protrusion 116 out of the state of abutting against each other.

As will be apparent by comparing with Fig. 5, while the arm 208 has been moved further forward, the connector 212 is unable to move forward due to the abutment between the protrusions 226 and the protrusion 116 unless the abutment is released. Fig. 6 shows the state immediately after the state of abutment has just been removed, and therefore the position of the connector 212 is substantially not different from that in the state of being unable to move forward. Since the spring 210 is compressible, it is more compressed in the state shown in Fig. 6 than in the state shown in Fig. 5, and the base 206 has been moved further forward. It is noted that in the shown embodiment, the protrusion 226 is provided on the connector 212, although the protrusion may also be provided on the lancet body 216.

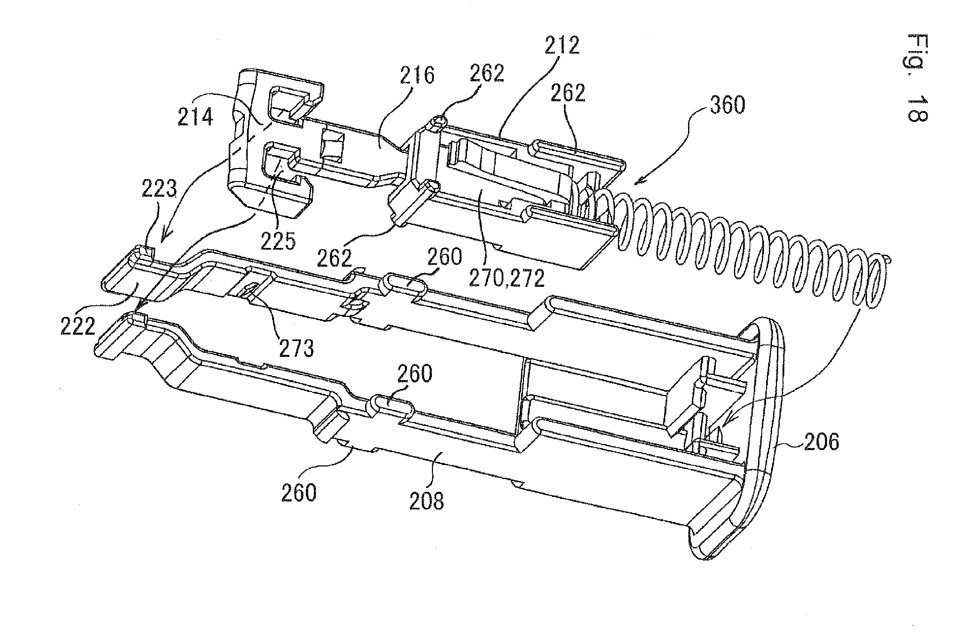

As will be easily understood from Fig. 3, Fig. 4, Fig. 5, Fig. 6 and Fig. 7, the lancet assembly of the present invention preferably has such a constitution that the front end portions of the arms can engage with the lancet cover. More specifically, the front end portions 222 of the arms 208 have hooked portions (or L-shaped portions) 223 which are bent outward, and the lancet cover 214 has portions 225 provided on the side thereof which engage with the hooked portions. In the shown embodiment, such portion defines a complementary space 225 into which the hooked portion 223 is to be fitted (refer to Fig. 18).

When the front end portions of the arms engage with the lancet cover as described above, the abutment between the front end portions 222 of the arms 208 and the lancet cover 214 is maintained by such engagement even after the pricking element 220 has been separated from the lancet cover 214. As a result, in the state shown in Fig. 5 where the distal end portion 232 of the pricking element is once exposed, the lancet cover 214 can move backward together with the arm 208 even when the force that pushes the base 206 is inadvertently removed and the arms 208 are moved back somewhat by the action of the spring 210. In a case wherein such an engagement cannot be maintained, the arms moving back bring the lancet cover in a free state, and it may become difficult to ensure the abutment between the front end portions 222 of the arms 208 and the lancet cover 214 when the arms are moved forward thereafter. It needs not to say that a form other than the hooked shape may be used as long as the front end portion of the arm can engage with the lancet cover.

What is to be noted in the lancet assembly of the present invention is that the lancet holder has guiding means provided on the side inner walls in the front end portion thereof, while the lancet cover has guided means which are guided by the guiding means. As a result, when the guiding means and the guided means cooperate with each other, the lancet cover moves obliquely forward (obliquely upward or obliquely downward) upon being pushed forward of the lancet cover which has been separated by the arms which are moved forward. More specifically, the lancet holder has, as the guiding means, sliding portions that extend obliquely forward on the side inner walls in the front end portion thereof, and the lancet cover has, as the guided means, portions such as protrusions that slide over the sliding portions.

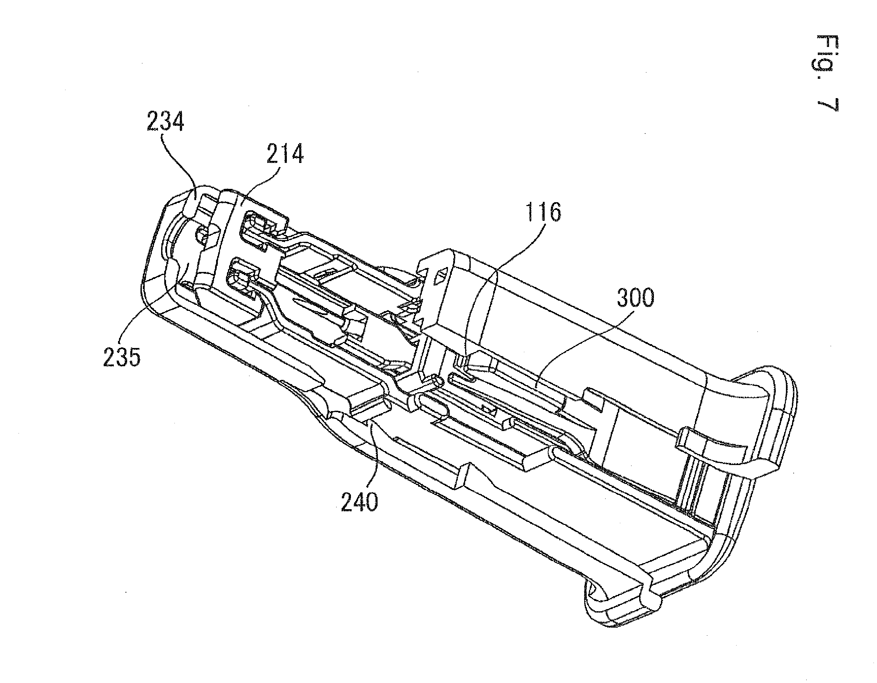

In the embodiment shown in Fig. 7, after undergoing the states shown in Fig. 4, Fig. 5 and Fig. 6, the lancet cover 214 which has been separated is pushed by the arms 208 to move, so as to be located at a position separated by a small gap 235 from the inside of the front wall 234 of the lancet holder (in other embodiment, the lancet cover may abut against the inside). In this state, it is ready to launch the lancet body with the distal end portion of the pricking element exposed.

Provided as the guided means on the sides of the lancet cover 214 are the tapered portions 236, of which width in the vertical direction decreases forward.
Provided on the side inner walls of the front end portion of the lancet holder 100 are guiding means 238 of tapered portions 238 of which width increases (or flares) toward the front (namely reverse tapered portion) and have sloped surfaces 254 that define the reverse tapered portions (refer to, for example, Fig. 3). The bottom surface of the tapered portion 236 of the lancet cover is adapted to slide obliquely upward on the sloped surface 254.

It is noted that Fig. 5 shows a state immediately before such sliding motion begins, while Fig. 6 shows a state after such sliding motion has begun. As a result, the tapered portions 236 of the lancet cover 214 that are pushed forward by the arms 208 move upward along the sloped surfaces 254 of the reverse tapered portion 238. As a result, the lancet cover 214 is located at a position higher than that shown in Fig. 5.

In Fig. 7, the lancet cover 214 has been moved further by the arms 208 forward obliquely upward, and is located adjacent to the inside of the front wall 234 of the lancet holder. This adjacent state is maintained by keeping the state of being pressed by the arms 208. It is noted that in the embodiment shown in the drawing, the lancet cover has moved obliquely and then moved a little forward, since the guiding means 238 has a horizontal portion 255 in front of the sloped surface.

It is necessary that the movement of the lancet cover 214 in the oblique direction as described above must be enough to ensure the distal end portion 232 of the pricking element which is exposed to move through the opening 108 positioned at the front end of the lancet holder 100 and prick a finger tip or the like. That is, when the lancet body 216 in which the distal end portion 232 of the pricking element is exposed is launched, the lancet cover 214 does not exist in front of the distal end portion 232 in the direction of movement thereof, so that the lancet cover 214 does not make contact with the distal end portion 232 so as not to impede the movement of the distal end portion (the lancet cover 214 does not exist in the trajectory of the distal end portion 232). The term "directly" was used previously in this sense.

When the lancet cover 214 is held the upper inside of the front end portion of the lancet holder 100, preparation for the pricking operation is completed. In this case, the taper-like shape protrusions 240 of which width in the horizontal direction increase (or flare) toward the distal end is provided on the side inner walls of the lancet holder (between the protrusion 228 described above and the front end portion) similarly to that described previously, so that in the course of inserting the lancet structure 200, wings or protrusions 230 which are provided on the arms 208 can get over the protrusions 240. The protrusion 240 is positioned preferably at such a position that the lancet cover 214 just comes to adjoin the inside of the front wall 234 of the lancet holder, when the wing or the protrusion 230 has got over the protrusion 240, as shown in the drawing. In other embodiment, it is also preferable that the adjoining state is achieved immediately before the wing gets over the protrusion 240 and the lancet is launched at the same time as getting over the protrusion (the state shown in Fig. 7).

With the wing or the protrusion 230 and the protrusion 240 provided as described above, descriptions similar to the descriptions given previously with reference to the protrusion 228 are applicable also to them.

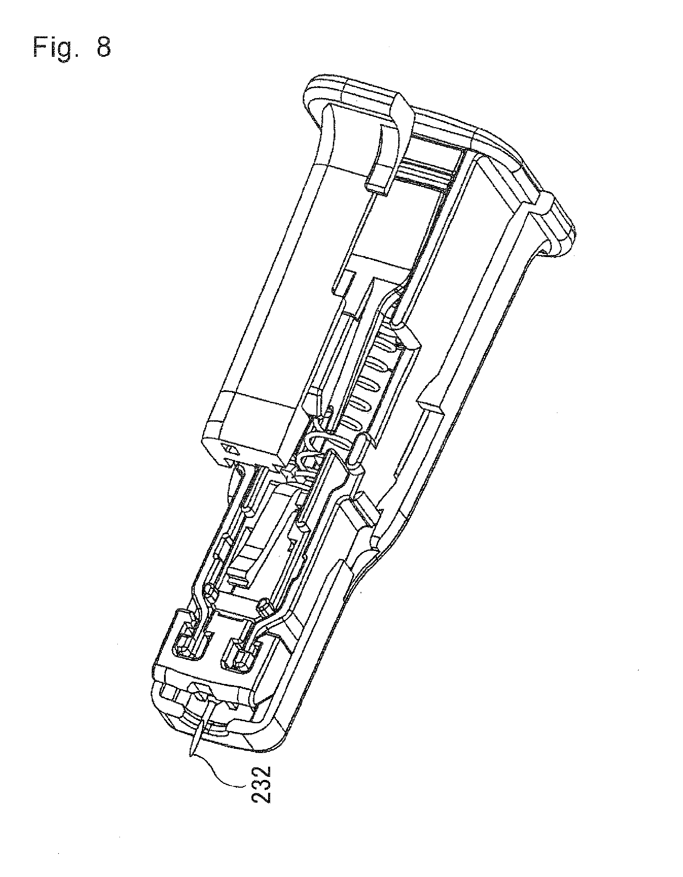

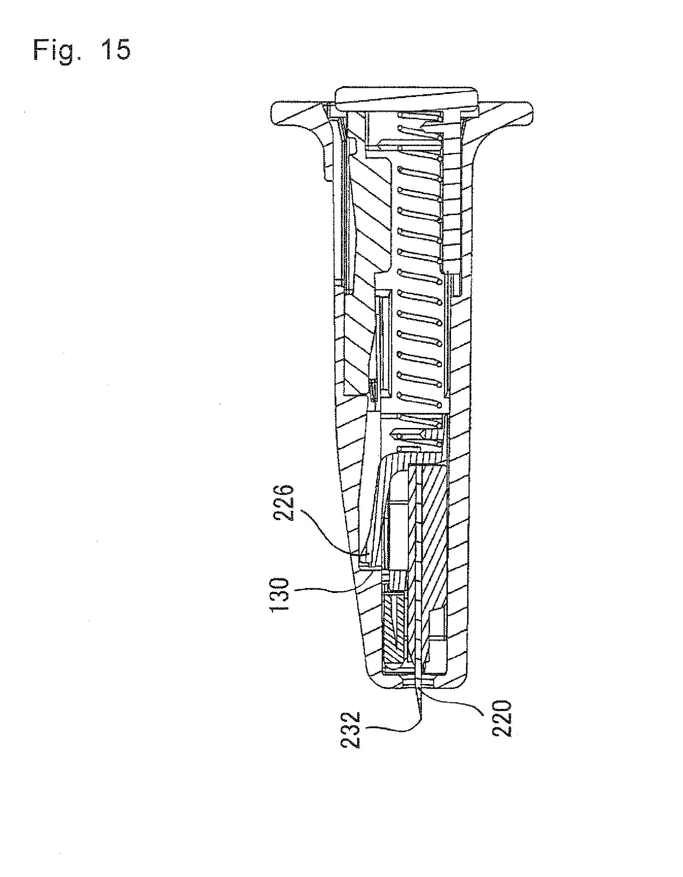

The preparation for the pricking operation is completed in the state shown in Fig. 7, namely in the state where nearly abutting is achieved between the protrusion 116 and the protrusion 226. As will be apparent, in the state of being ready for pricking, the protrusion 226 of the connector 212 to which the lancet body 216 is connected remains abutting against the protrusion 116 of the lancet holder which is positioned in front of the trigger 300. Since the protrusion 226 is gradually pressed downward (refer to the arrow A in Fig. 13) as described previously, the region over which both protrusions are abutting against each other decreases definitely, and slight abutment is maintained. When the trigger is moved further forward from the state shown in Fig. 7, the protrusion 226 moves further downward so that the abutting region eventually diminishes. That is, the forces of the protrusion 116 and the protrusion 226 pressing each other are brought completely out of the alignment, thus breaking the engagement and reaching the state shown in Fig. 8 and Fig. 15.

As a result, the compressed spring 210 tries to restore its original shape, so that the connector 212, and therefore the lancet body 216 having the prick element of which distal end portion is exposed is launched forward. Since the distal end portion 232 can protrude from the opening 108 without being hampered by the lancet cover 214, the pricking operation can be achieved. The state wherein the distal end portion is protruding is shown schematically in the perspective view of Fig. 8. It is noted that the spring 210 is instantaneously released from the state of being compressed, and is therefore elongated in the state shown in Fig. 8 in comparison to the state shown in Fig. 2.

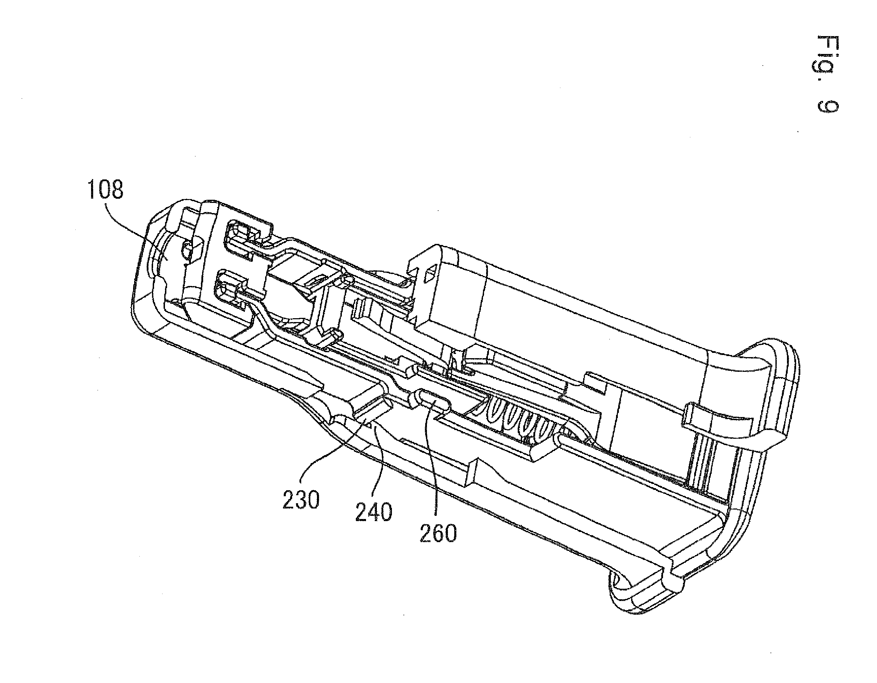

When the distal end portion 232 of the pricking element protrudes through the opening 108, while the predetermined position is prickd, the protrusion 226 of the connector 212 collides with the wall 130 that serves as an abutting portion provided on the front inside portion of the side surface of the lancet case. As a result, the spring 210 which has expanded is caused to restore the original shape by the reaction so as to finally return to the shape similar to that in the state shown in Fig. 2. This state is shown in Fig. 9. In the state where the spring 210 has restored the original shape, the distal end portion 232 of the pricking element is positioned in the inside at a sufficient distance from the opening 108 of the lancet holder (the distal end portion is located below the lancet cover 214 and therefore it is not seen in Fig. 9). As a result, it is made possible to effectively avoid such a situation that the distal end portion 232 of the pricking element that is exposed is touched through the opening 108 from the outside of the lancet holder 100.

Fig. 9 shows the state where the pricking operation by the lancet assembly has been completed, and therefore the assembly may be disposed of in the state shown in Fig. 9. As described previously, providing the protrusions 230 and 240 having the wing or tapered configuration makes it impossible to draw the lancet structure 200 from the lancet holder 100 in the state shown in Fig. 9. As a result, the distal end portion of the pricking element will never be exposed even when the assembly is discarded in the state shown in Fig. 9. Therefore inadvertent contact with the distal end portion can be avoided and safety of disposal is improved.

The state shown in Fig. 9 corresponds to the state after the lancet has been launched. Since the wings 230 are engaged with the protrusions 240, it is difficult for the lancet structure to retract backward from the position shown in Fig. 9 within the lancet holder 100. Therefore, it is difficult to pull out the lancet structure, of which the pricking element 220 is exposed, from the lancet holder 100 of the used lancet assembly.

In a preferred embodiment, each of the arms 208 has the protrusion 260 as a guide pin (or guide bar) as shown in the drawing. The guide pin is adapted so as to cooperate with a channel that is provided on the inner surface of the lancet holder so as to extend in the pricking direction. That is, when the lancet structure is inserted into the lancet holder, the guide pins slide in the channels so that the arms move forward smoothly within the lancet holder, thus making the insertion smoother. Thus the guide pins guide the arms in moving forward in the lancet holder. Such guide pin may be provided on the upper side and/or the lower side of the arm.

In other preferred embodiment, the connector 212 has a protrusion 262 as a guide pin (or guide bar) as shown in the drawing. The guide pin cooperates with other channel which is provided on the inner surface of the lancet holder and extends in the pricking direction. That is, when the lancet structure is inserted into the lancet holder, the guide pin moves sliding in the channel so that the lancet body moves forward smoothly in the lancet holder, thereby making the insertion more smooth. The channel also makes it smoother for the lancet body 216 to move back and forth in the pricking direction within the lancet holder when the lancet body 216 is launched, pricking operation is carried out, and the distal end portion of the pricking element is retracted thereafter. In other words, the guide pin guides launching of the lancet body that has the exposed pricking element. The guide pin may be provided on the upper side and/or the lower side of the arm.

The lancet structure of the present invention is constituted from the ejector 202 and the lancet 204, which are connected integrally via the connector 212 and the spring 210. These members may be connected by any proper means. For example, of a set of a recess (female portion or key slot) and a protrusion (male portion or key) which can be fitted with each other (preferably having shapes complementary to each other), one of the recess and the protrusion is formed on one of the members to be mated and the other one is formed on the other one of such members. When forming the recess and the protrusion, the recess is formed on the connector and the protrusion is formed on the lancet body (or vice versa) such that the lancet body and the connector are engaged with (or separated from) each other in the vertical direction, and cannot be separated in the front and back direction.

An example of such a connection is shown in Fig. 18. In Fig. 18, the lancet body 216 has a protrusion 270 as a whole, and the connector 212 has a space 272 as the recess into which such protrusion can be fitted. The space 272 is occupied by the protrusion 270, and therefore the elements 270 and 272 are shown as the same element. As will be easily understood, when the protrusion 270 is moved upward from below the connector 212 and fitted in the space 272, the connector 212 and the lancet body 216 are not separated from each other by the force acting in the front-to-back direction, and they move together. When a force acts in the vertical direction, however, the connector 212 and the lancet body 216 can be easily separated from each other.

It is of advantage to employ such a connection manner as described above, since it enables it to form the lancet and the ejector separately and combine them into an integrated member. The connector 212 and the lancet 204 that have been connected as described above are fitted from the above and connected to the spring 210 so as to obtain the assembly 360 as illustrated, and the assembly 360 is fitted from the above as indicated by the arrow and connected to the integrated member of the arm and the base so as to obtain the lancet structure.

In this specification, expressions of "taper off" and "flare" as to the tapered portion mean that a width of the tapered portion (a dimension perpendicular to the forward direction) decreases and increases, respectively, when viewed in a forward direction (the direction toward the "forward" in Fig. 1). The "reverse tapered portion" means a configuration of having a tapering shape reverse to the corresponding tapered portion. That is, when one tapers off and the other flares, the former may be called the tapered portion and the latter called the reverse tapered portion, or the former may be called the reverse tapered portion and the latter called the tapered portion. The same applies also to the "taper-like shape."

The pricking operation by means of the lancet assembly of the present invention is carried out, for example, in the following procedures:
1) First, the lancet structure 200 is inserted into the lancet holder 100 through the opening 104 positioned at the rear end thereof, so as to expose the pushbutton 117 of the trigger 300 through the opening 120 provided in the side surface of the lancet case (the state shown in Fig. 3 achieved through the action indicated by the arrow in Fig. 1).

2) While pressing the pushbutton 117 into the lancet case inside, the lancet structure 200 is moved forward in the lancet holder 100, and the front end of the protrusion 226 provided on the connector 212 or on the lancet body 216 (the protrusion is provided on the connector in the embodiment shown) is brought abutment against the rear end of the protrusion 116 serving as a stopper provided on the trigger counterpart 110 of the lancet holder 100, thereby to stop the movement of the lancet 204 and prevent it from moving further forward. That is, the lancet is restricted in its forward movement. In addition, with the lancet 204 engaged, the base 206 is pushed further forward to start compressing the spring 210 from its unloaded state, so that the spring 210 stores energy (the state between those shown in Fig. 3 and Fig. 4).

3) As the base 206 is further pushed into the lancet holder 100, the arm 208 pushes the lancet cover forward, so that breakage occurs in the notch 218 (which functions as a weakened portion) that connects the lancet cover 214 and the lancet body 216, and the lancet cover 214 and the lancet body 216 are separated from each other (the state shown in Fig. 4). Then, when the arm 208 is moved further forward, the distal end portion 232 of the pricking element is exposed (the state shown in Fig. 5). It is noted that it is preferable to provide the protrusions 280 on the lancet body, in order to prevent the lancet body 216 from deforming when the lancet cover 214 is separated.

4) When the base 206 is pushed so as to move the arm 208 further forward, the lancet cover 214 moves forward obliquely upward, and is held in abutting against the inside of the wall 234 at the front end of the lancet holder 100 (the state shown in Fig. 7 via the state shown in Fig. 6).

5) When the base 206 is pushed further so as to move the portion near the front end portion 302 of the trigger 300 forward in the front of the lancet holder 100, the protrusion 116 and the protrusion 226 are released from their abutment state (the state immediately after that shown in Fig. 7), the spring 210 which has been compressed instantaneously expands, so as to launch the lancet body 216 and the distal end portion 232 of the pricking element protrudes through the opening 108 and carry out the pricking operation (the state shown in Fig. 8).

6) Then, the spring 210 restores its original shape, and the distal end portion 232 of the pricking element retracts sufficiently inside from the opening 108 (the state shown in Fig. 9).

In a preferred embodiment, as will be understood from Fig. 18, the arm 208 may have the protrusion 273 on the inside thereof. It is preferable that, when the connector 212 passes along the side of the protrusion, the protrusion protrudes from the arm toward the inside to such an extent as it can barely contact the connector. This has such a function to reduce the possibility of the second time pricking operation in that the lancet body which has retracted as shown in Fig. 9 after pricking moves forward again by the expansion and contraction vibration of the spring, and protrudes through the opening 108 again thereby.

It is noted that although such a contact occurs in the first time pricking action, this contact does not substantially hamper the movement of the connector for pricking operation, since the spring 210 expands with a strong force. However, for the connector that is urged to move forward again by the vibration after the pricking element has once retracted, the slight contact described above generates significant resistance against the forward movement, thus making the second time pricking substantially impossible.

The operation of applying to the portion (for example, a finger tip) from which a blood sample is to be taken onto the opening 108 positioned at the front end of the lancet holder may be done in any stage as long as it is before the lancet body 216 is launched. With the case of the lancet assembly of the present invention, the procedures from 2) to 5) can be carried out continuously by pushing the base in a stroke while pressing the pushbutton 117 after disposing the lancet structure within the lancet holder to achieve the state shown in Fig. 3. Therefore, the applying operation of the portion to be pricked may be done immediately before the steps 3) after providing (for example, commercially supplying) the assembly in the state of step 2).

In order to make it easier to understand the positional relationship between the lancet structure and the lancet holder of the present invention or the positional relationship between the components that form the lancet structure and the lancet holder, Fig. 10 to Fig. 16 show the lancet holder in the states shown in Fig. 3 to Fig. 9, respectively, in schematic cross sectional views along the central line of the lancet holder (line X-X in Fig. 3).

Fig. 10 is a cross sectional view corresponding to the state shown in Fig. 3. Fig. 10 shows the front end portion 302 of the pushbutton 117 of the trigger 300 which abuts against the wall surface that defines the front end portion 122 of the opening 102 provided in the upper side surface 101 of the lancet holder 100. It can be seen that the protrusion 226 of the connector 212 abuts against the protrusion 116 positioned in the rear portion of the trigger counterpart 110 of the lancet holder 100. The lancet cover 214 and the lancet body 216 are integrally connected via the notch 218.

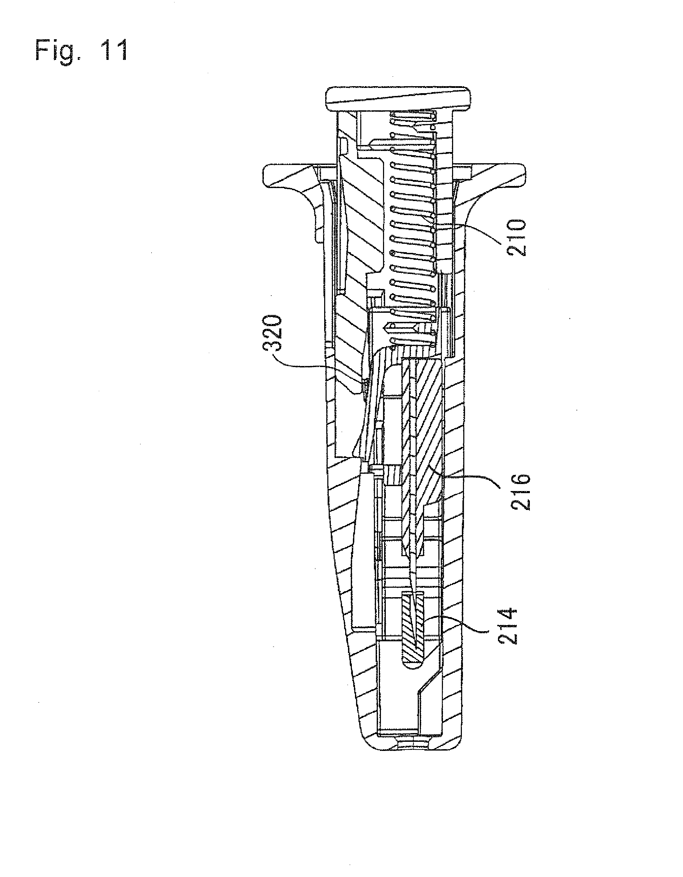

Fig. 11 is a cross sectional view corresponding to the state shown in Fig. 4. In Fig. 11, the lancet cover 214 is separated from the lancet body 216. It can be seen that the base 206 is further pushed and the spring 210 is compressed in comparison with the state shown in Fig. 10. The pushbutton 117 of the trigger has moved forward in contact with the inside of the upper side surface of the lancet case 100.

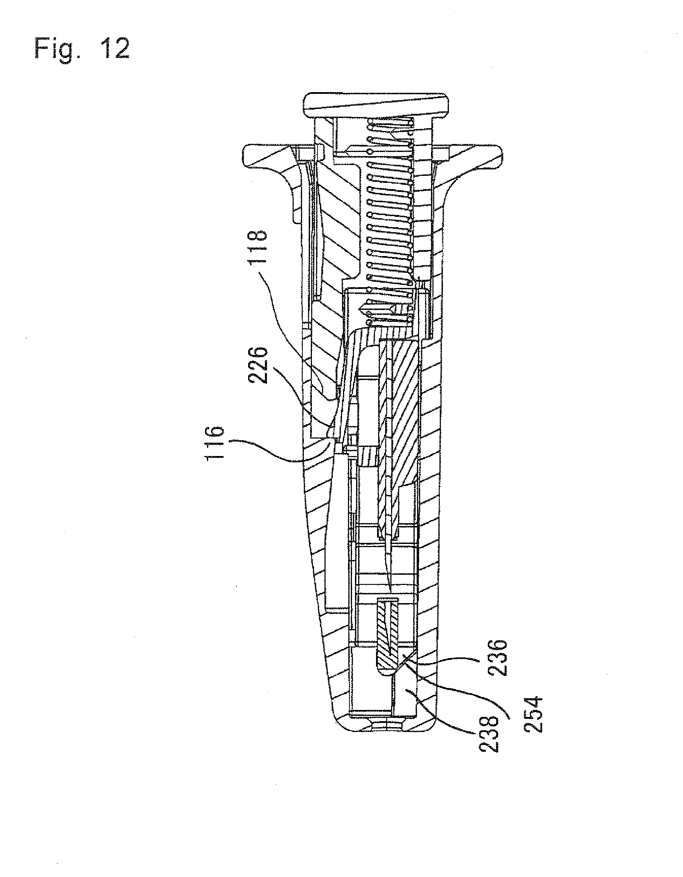

Fig. 12 is a cross sectional view corresponding to the state shown in Fig. 5. In Fig. 12, the lancet cover 214 is separated completely from the pricking element 220 so as to expose the distal end portion thereof. The spring 210 is compressed further in comparison to the state shown in Fig. 13.

As will be understood from Fig. 12, the front end portion 302 of the trigger 300 is in the state immediately before making contact with the protrusion (specifically the sloped surface thereof) 226 which protrudes forward obliquely upward from the connector 212 and which is abut against the protrusion 116 positioned at lower rear portion of the trigger counterpart 110. The tapered portion 236 serving as the guided means of the lancet cover is in the state immediately before it abuts against the guiding means 238 that is provided on the inside of the front end wall of the lancet case. Therefore, when the base is further pushed from this state, the front end portion 302 abuts against the protrusion 116 and then gradually presses the protrusion 226 downward while the arm moves forward so that the lancet cover moves up on the sloped surface and achieves the state shown in Fig. 6 and Fig. 13.

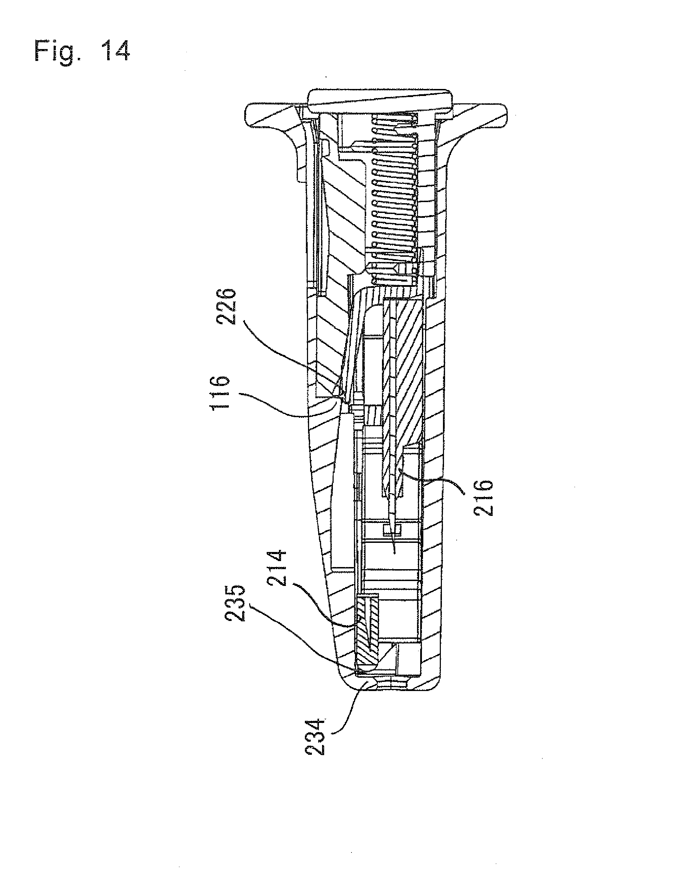

Fig. 13 is a cross sectional view corresponding to the state shown in Fig. 6. In Fig. 13, the lancet cover 214 is in the course of being moved forward obliquely upward by the guiding means of the lancet cover 214. This is because the tapered portions 236 located on the sides of the lancet cover moves along the sloped surfaces 254 of the guiding means 238 provided on the inside of the front wall of the lancet holder. In this state, with the front end portion 302 of the trigger having moved forward while pressing the protrusion 226 further downward (refer to the arrow), the protrusion 116 located in the lower rear portion of the trigger counterpart 110 and the protrusion 226 that protrudes forward obliquely upward from the connector 212 are a little before being released from abutting against each other. From this state, the state shown in Fig. 7 and Fig. 14 is achieved.

Fig. 14 is a cross sectional view corresponding to the state shown in Fig. 7. In Fig. 14, the front end portion 302 of the trigger is moved further forward so as to push the protrusion 226 further downward to the state immediately before the protrusion 116 located at the lower rear portion of the trigger counterpart 110 and the protrusion 226 that protrudes forward obliquely upward from the connector 212 are released from their abutment against each other. As will be seen from the drawing, the lancet cover 214 has just fully moved forward obliquely upward, and therefore the lancet cover 214 does not impede the movement of the pricking element at all.

Fig. 15 is a cross sectional view corresponding to the state shown in Fig. 8. In the state shown in Fig. 14, the trigger has been moved further forward to cancel the state of abutment, and the spring 210 has instantaneously expanded. In this state, the connector 212 has been moved forward and hit the abutting portion 130 provided on the inner wall of the lancet holder, and the distal end portion 232 of the pricking element has protruded through the front end opening 108 of the lancet holder 100. Since the portion to be pricked is applied to the front end opening 106 of the lancet holder 100, the pricking operation is carried out at this time.

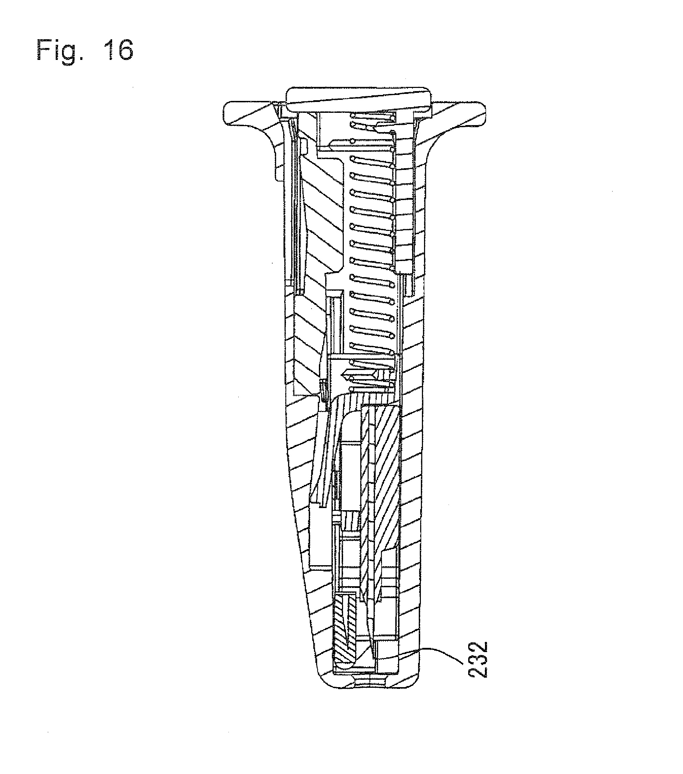

Fig. 16 is a cross sectional view corresponding to the state shown in Fig. 9. In the state shown in Fig. 16, the distal end portion 232 of the pricking element has been caused, by the reaction upon pricking and the reaction upon the collision of the protrusion against the wall 130, to retract inward at a sufficient distance from the front end opening 108 of the lancet holder 100. As will be seen from the drawing, it is quite difficult to touch the distal end portion 232 of the pricking element through the opening 108, and is substantially impossible unless it is deliberately attempted.

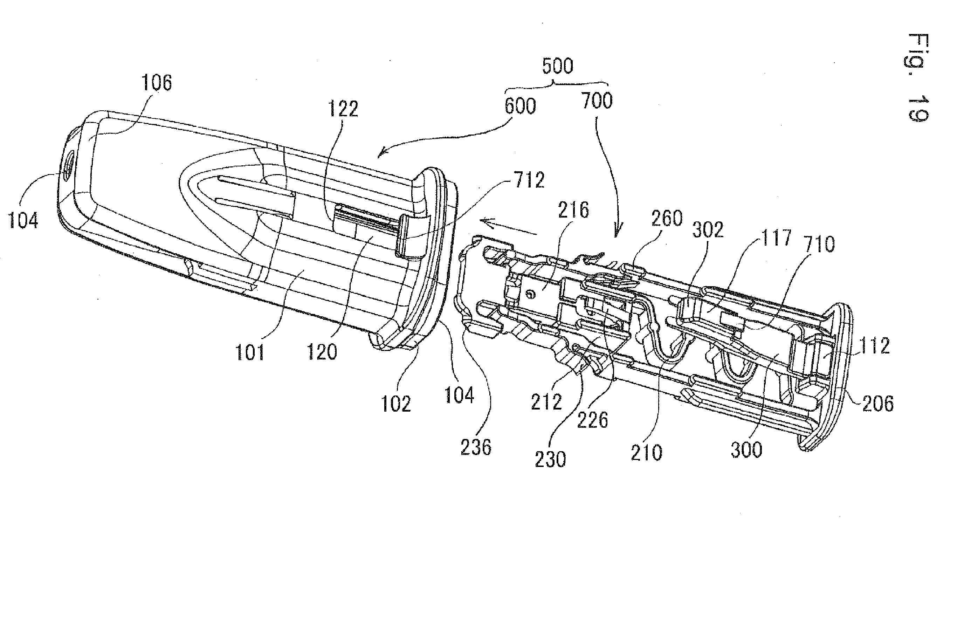

A lancet assembly 500 of the present invention is shown in a schematic perspective view of Fig. 19. The lancet assembly 500 corresponds to the lancet assembly of said "another preferred embodiment" described above (it is noted that it has a flap that extends from the rear of the pushbutton backward), and is constituted from a lancet holder 600 and a lancet structure 700. In the state shown in the drawing, the lancet structure 700 is immediately before being inserted in the direction indicated by the arrow into the inner space of the lancet holder 600.

The lancet assembly 500 shown in the drawing may be similar to the lancet assembly of said "one preferred embodiment" described above except that the pushbutton 117 has the flap 710 that extends from the rear portion 708 thereof. Accordingly, the elements (or members) of the lancet assembly 500 which have the same functions as the elements of the lancet assembly 10 are identified with the same reference numerals as those of the lancet assembly 10 having the same functions. It is noted that the dimensions and/or the shapes of these elements may be different from those of said "one preferred embodiment", and also they may differ in said "another preferred embodiment" which is shown in the drawings. In the embodiment shown in the drawing, the spring 210 is a resin spring which is connected to the base 206, specifically to the portion where the trigger 300 is to be fitted in the base 206.

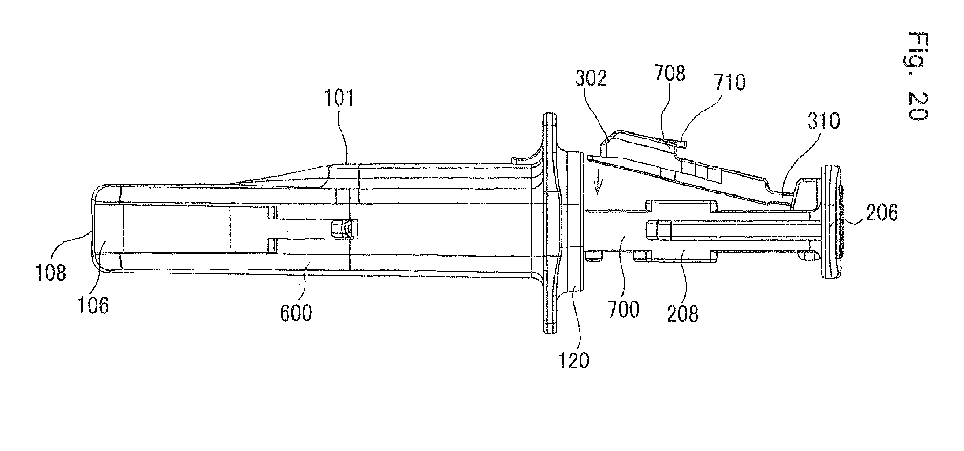

Fig. 20 schematically shows a state of starting the insertion from the state shown in Fig. 19, when viewed sideways. As will be understood from Fig. 20, the portion located ahead of the connector 212 of the lancet structure is disposed in the lancet holder. The trigger 300 extends from the base 206 forward obliquely upward. When it is attempted to insert the lancet structure 700 further, it cannot enter the opening 104 of the lancet holder in the state as shown in the drawing since the front end portion 302 of the trigger 300 is located at a high position. As a result, the front end portion of the trigger is pressed down to temporarily bring the position thereof a little lower than the state shown in the drawing as indicated by the arrow so as to insert into the lancet holder. In order to makes it easier to change the position of the front end portion of the trigger as described, the trigger is formed from a resin and a portion of the trigger 300 proximate to the base 206 is formed as a thinned portion 310.

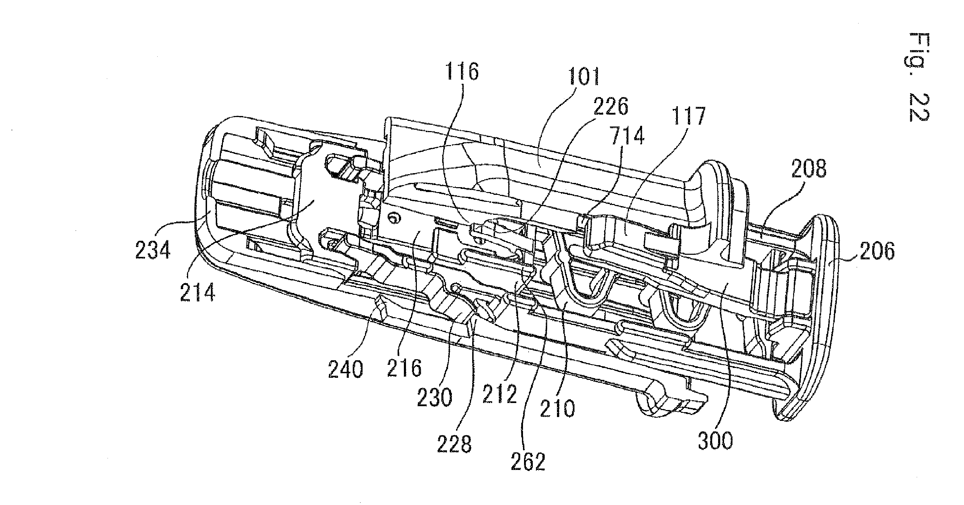

Fig. 21 is a schematic perspective view of the lancet assembly 500 according to said "another preferred embodiment" of the present invention, showing a state in which the insertion of the lancet structure 700 into the lancet holder 600 has been completed, thereby completing the assembly. In the embodiment shown in the drawing, a flap 710 extends backward while getting over the wall (or edge) that defines the rear end portion of the opening 120 provided on the side surface of the lancet holder. The state of the inside of the lancet assembly shown in Fig. 21 is shown in a perspective view in Fig. 22 similarly to that of Fig. 3.

The state shown in Fig. 21 is achieved by inserting the lancet structure 700 as shown in Fig. 20, and then pushing the base 206 further while the front end portion 302 of the trigger 300 is pressed down as indicated by arrow so as to elastically deform downward. When pushing in this way, the connector 212, specifically the front end portion of the protrusion 226 that extends therefrom forward and obliquely abuts against the protrusion 116 that protrudes toward the inside of the lancet holder as shown in Fig. 22, thereby achieving the state of engagement in which the connector 212, hence the lancet body 216 connected thereto is prevented from moving forward. Then, when the base 206 is pushed in further, the spring 210 is compressed a little. Then, the pushbutton 117 is exposed through the opening 120 provided in the side surface of the lancet holder, and preferably protrudes from the side surface.

For this protruding action, in case the trigger 300 is formed from, for example a resin to extend obliquely upward with respect to the direction of inserting the lancet structure 700 as shown in Fig. 20 when the trigger is elastically pressed down as described above, the trigger 300 tries to move to return to its original shape of extending obliquely upward. Therefore, as the lancet structure is inserted as described above and the pushbutton is located below the opening 120 provided in the side surface of the lancet holder (more strictly, as the rear end portion of the flap 710 passes below the rear end of the opening 120), the pushbutton is automatically exposed through the side surface of the lancet holder, preferably protrudes.

Then, when the force that pushes the base 206 is removed, since the spring 210 that has been compressed as described above expands a little, the pushbutton retracts to move away from the front end portion 122 of the opening provided in the side surface of the lancet holder (accordingly a space 714 is formed between the front end portion 302 of the pushbutton and the wall or the edge that defines the front end portion 122 of the opening), and the flap 710 gets over the rear end wall or the edge that defines the opening of the side surface of the lancet holder 700 and extends backward, namely the state shown in Fig. 21 is achieved.

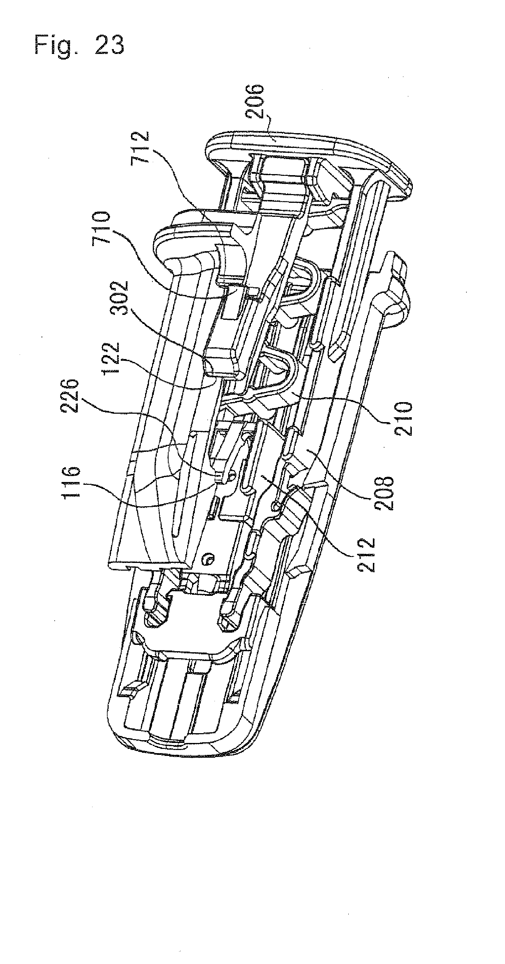

When the lancet assembly of the present invention shown in Fig. 21 is used, a force acting forward is applied to the base 206 so as to move the flap 710 from the edge 212 forward, into the state shown in Fig. 23. The state shown in Fig. 23 is substantially equivalent to the state shown in Fig. 3, in which the front end portion 302 of the pushbutton is abutting against the wall or the edge 122 that defines the front end portion of the opening 120 of the side surface of the lancet holder 600. Therefore, after the state shown in Fig. 23 has been achieved, the lancet assembly 500 of said "another preferred embodiment" can be operated similarly to the lancet assembly 10 of said "one preferred embodiment" of the present invention.

As described above, the lancet assembly 500 of said "another preferred embodiment" has the flap 710. As a result, even when an extraneous force is applied downward to the pushbutton 117, the pushbutton is prevented from its movement to the inside of the lancet holder unless the pushbutton moves forward and the flap 700 comes away the wall or the edge that defines the rear end of the opening. When such a force is applied inwardly to the pushbutton in the lancet assembly of said "one preferred embodiment" of the present invention shown in Fig. 3, the pushbutton moves downward.

As described above, the flap 700 is capable of preventing the pushbutton and therefore the trigger from being displaced downward. For example, when the completely assembled lancet assemblies are piled up in the process of manufacturing the lancet assembly of the present invention shown in Fig. 3 and/or the process of storing are piled up and, as a result, the pushbutton is loaded with the weight of the other lancet assemblies for a long period of time, there is a possibility that the trigger, that should normally extend forward obliquely upward, does not protrude from the opening in the side surface due to creeping that is characteristic to resin products. Providing the flap has an advantage of avoiding such a possibility.

The lancet assembly of the present invention has been described with reference to the accompanying drawings. However, the shape of the cross section of the lancet assembly perpendicular to the direction of pricking needs not to be a rectangle, an elongated general rectangle or an elongated polygon as shown, and may be an elongated circle, an oval, a circle, a polygon or any other shape as required. For example, in the lancet assemblies of the embodiments shown in Fig. 1 to Fig. 18, the cross section may be proximate to a circle with the spring being turned 90° around the direction of pricking and connected to the connector and the base.

### Industrial Applicability

The lancet assembly of the present invention described above provides means for taking a blood sample more easily.

## Claims

1. A lancet assembly comprising a lancet structure and a lancet holder which holds the lancet structure, in such a constitution that by pressing the lancet structure into the lancet holder, the following steps are carried out: the step of separating a lancet cover from a lancet body so as expose a distal end portion of a pricking element, and then the step of launching the lancet body having the distal end portion of the pricking element exposed so as to prick a predetermined portion,
**characterized in that**
a trigger used to launch the lancet body has a pushbutton exposed through an opening provided in a side surface of the lancet holder,
the lancet body of which the distal end portion of the pricking element is exposed is launched by moving the trigger forward while the exposed pushbutton is pressed into the lancet holder.

2. The lancet assembly according to claim 1 **characterized in that** a front end portion of the pushbutton is abutting against with a wall that defines the front end portion of the opening of the side surface in the state of the pushbutton being exposed.

3. The lancet assembly according to claim 1
**characterized in that** a front end portion of the pushbutton is separated from the wall that defines the front end portion of the opening of the side surface in the state of the pushbutton being exposed.

4. The lancet assembly according to claim 3 **characterized in that** the pushbutton has a flap that protrudes from a rear portion thereof backward, and the flap is disposed above a wall that defines a rear end portion of the opening of the side surface in the state of the pushbutton being exposed.

5. The lancet assembly according to any one of claims 1 to 4 **characterized in that** the pushbutton is in the form of an elongated shape, and it extends obliquely relative to a direction in which the side surface of the lancet holder.

6. The lancet assembly according to any one of claims 1 to 5 **characterized in that** at least a portion of the trigger can elastically deform.

7. The lancet assembly according to any one of claims 1 to 6 **characterized in that** the pushbutton has a portion which protrudes out through the opening in the side surface of the lancet holder when it exposed through the opening.

8. The lancet assembly according to any one of claims 1 to 7 **characterized in that** a front end portion of the pushbutton defines a sloped surface which extend downward toward its front.

9. The lancet assembly according to any one of claims 1 to 8 **characterized in that** the trigger has an extension portion along its side.

10. The lancet assembly according to any one of claims 1 to 9 **characterized in that**
the lancet structure is composed of an ejector and a lancet;
the ejector comprises a trigger, an arm, a spring and a base onto which the trigger, the arm and the spring are attached, and the spring has a connector provided on its front end and is connected to the base at its rear end;
the lancet comprises a lancet body, a lancet cover and a pricking element with the pricking element being in the lancet body and the lancet cover while straddling across the lancet body and the lancet cover, and the distal end portion of the pricking element is enclosed by the lancet cover;
the lancet body is connected to the connector;
the lancet holder has, at the front end thereof, an opening through which the distal end portion of the pricking element passes; and
when the lancet structure is inserted in the lancet holder and the base is moved relatively toward the connector, and the spring is compressed with the connector being engaged with the lancet holder, the lancet cover is removed from the pricking element and the distal end portion of the pricking element which has been enclosed is exposed, and then when the movement of the base is further continued, the trigger releases the lancet body from the engagement state.

11. The lancet assembly according to claim 10
**characterized in that**
the lancet cover is located in front of the arm, and
when the base is moved relatively toward the connector so as to compress the spring while keeping a front end of the arm abutting against a rear end of the lancet cover, the lancet cover and the lancet body are separated at a weakened portion.

12. The lancet assembly according to claim 10
**characterized in that**
the lancet cover is positioned in front of the arm, and
when the base is moved relatively toward the connector so as to compress the spring while keeping the front end of the arm abutting against the rear end of the lancet cover, the lancet cover gets away from the lancet body.

13. The lancet assembly according to any one of claims 10 to 12 **characterized in that** the front end of the arm engage with the lancet cover.

14. The lancet assembly according to claim 13 **characterized in that** the front end of the arm has a hooked portion (or L-shaped portion) that is bent inwardly, and the lancet cover has, on the side thereof, a portion that engages with the hooked portion.

15. The lancet assembly according to any one of claims 10 to 14 **characterized in that** the separated lancet cover moves forward and obliquely by means of the arm which moved forward, so that the opening located at the front end of the lancet holder is in front of the exposed pricking element.

16. The lancet assembly according to any one of claims 10 to 15 **characterized in that** the lancet body comprises a protrusion,
upon compressing the spring, the protrusion of the lancet body abuts against a protrusion of the lancet holder, so that the connector is prevented from its moving forward, and
the trigger release such abutting state.

17. The lancet assembly according to any one of claims 10 to 16 **characterized in that** the base includes a pair of the arms, and the spring is located between the arms.

18. The lancet assembly according to any one of claims 10 to 17 **characterized in that** the connector, the lancet body or the spring includes a protrusion which extends forward and obliquely therefrom, and when the trigger which moves forward relative to such oblique direction contacts with the protrusion and is pushed forward, the connector, the lancet body or the spring is pushed downward to move so that the abutment state is finally released.

19. A lancet structure which is for the formation of the lancet assembly according to any one of claims 1 to 18.

20. A lancet holder which is for the formation of the lancet assembly according to any one of claims 1 to 18.

21. A kit of a lancet assembly composed of the lancet structure according to claim 19 and the lancet holder according to claim 20.
